# EUROPEAN PATENT SPECIFICATION

(11) **EP 3 907 504 B1**
(45) Date of publication and mention of the grant of the patent: **29.07.2026**
(21) Application number: 19907900.5
(22) Date of filing: 27.12.2019
(51) Int. Cl.: G01N 33/53, C07K 14/705

(54) **TEST METHOD FOR ULCERATIVE COLITIS AND PRIMARY SCLEROSING CHOLANGITIS**
TESTVERFAHREN FÜR COLITIS ULCEROSA UND PRIMÄRE SKLEROTISCHE CHOLANGITIS
MÉTHODE DE TEST POUR LA RECTOCOLITE HÉMORRAGIQUE ET LA CHOLANGITE SCLÉROSANTE PRIMITIVE

(30) Priority: 04.01.2019 JP 2019000060
(43) Date of publication of application: 10.11.2021
(73) Proprietor: Kyoto University, Kyoto-shi, Kyoto 606-8501 (JP)
(72) Inventor: SHIOKAWA, Masahiro, Kyoto-shi, Kyoto 606-8501 (JP); KUWADA, Takeshi, Kyoto-shi, Kyoto 606-8501 (JP); KODAMA, Yuzo, Kyoto-shi, Kyoto 606-8501 (JP); SENO, Hiroshi, Kyoto-shi, Kyoto 606-8501 (JP); CHIBA, Tsutomu, Kyoto-shi, Kyoto 606-8501 (JP)
(74) Representative: D Young & Co LLP
(86) International application number: PCT/JP2019/051592
(87) International publication number: WO 2020/141608

(56) References cited:
- JP-A- 2006 516 636
- JP-A- 2009 500 041
- JP-A- 2014 167 446
- MAHLER MICHAEL ET AL: "PR3-ANCA: A promising biomarker for ulcerative colitis with extensive disease", CLINICA CHIMICA ACTA, ELSEVIER BV, AMSTERDAM, NL, vol. 424, 24 June 2013 (2013-06-24), pages 267 - 273, XP028708564, ISSN: 0009-8981, DOI: 10.1016/J.CCA.2013.06.005
- DANESE, S. ET AL.: "Angiogenesis as a Novel Component of Inflammatory Bowel Disease Pathogenesis", GASTROENTEROLOGY, vol. 130, no. 7, 2006, pages 2060 - 2073, XP026190953, DOI: 10.1053/j.gastro.2006.03.054
- PENG, Z. W. ET AL.: "Integrin alpha V beta 6 Critically Regulates Hepatic Progenitor Cell Function and Promotes Ductular Reaction, Fibrosis, and Tumorigenesis", HEPATOLOGY, vol. 63, no. 1, 8 October 2015 (2015-10-08), pages 217 - 232, XP055723362
- MAHLER, M. ET AL.: "PR3-ANCA: A promising biomarker for ulcerative colitis with extensive disease", CLINICA CHIMICA ACTA, vol. 424, 24 June 2013 (2013-06-24), pages 267 - 273, XP028708564, DOI: 10.1016/j.cca.2013.06.005
- JUNFENG WANG, DAWEI SUN, YANBO WANG, FENGHAI REN, SAINAN PANG, DANDAN WANG, SHIDONG XU: "PR3-ANCA: A Promising Biomarker in Primary Sclerosing Cholangitis (PSC", PLOS ONE, vol. 9, no. 11, e112150, 1 January 2014 (2014-01-01), pages 1 - 7, XP055229063, ISSN: 1932-6203, DOI: 10.1371/journal.pone.0112150
- ZENG, D. F. ET AL.: "Autoantibody against integrin alphavbeta3 contributes to thrombocytopenia by blocking the migration and adhesion of megakaryocytes", JOURNAL OF THROMBOSIS AND HAEMOSTASIS, vol. 16, no. 9, 28 June 2018 (2018-06-28), pages 1843 - 1856, XP055723366

## Description

### Technical Field

An aspect of the present invention relates to a method for testing ulcerative colitis (UC) and use of a test kit for testing UC.

Another aspect of the present invention relates to a method for testing primary sclerosing cholangitis (PSC) and use of a test kit for testing PSC.

### Background Art

In the case of ulcerative colitis, erosive or inflammatory ulcers continuously develop from the rectum on the large-intestinal mucosa, and ulcerative colitis is associated with symptoms such as diarrhea, bloody stool, and abdominal pain. Ulcerative colitis most frequently occurs in young people, it also occurs in elderly people, and active phases and remission phases are repeated. Thus, long-term treatment is required. In recent years, ulcerative colitis is increasing throughout the world.

Primary sclerosing cholangitis (PSC) is a progressive chronic liver disease that involves multifocal or diffuse narrowing of the intrahepatic and extrahepatic bile ducts. Primary sclerosing cholangitis is considered as a multifactorial disease including immunological defects, although the cause thereof remains unknown. Primary sclerosing cholangitis is often complicated with an inflammatory bowel disease (ulcerative colitis, in particular).

Patent Literature 1 discloses a mapping method for diagnosis and/or prediction of prognosis of ulcerative colitis comprising classifying the intestinal flora of a subject into 10 intestinal bacterial groups and data-processing each of the intestinal bacterial groups by selforganizing map analysis.

Patent Literature 2 describes measurement of soluble LR11 concentration in the blood-derived sample obtained from a mammalian animal as an indicator for evaluating an extent of severity and prediction of prognosis of liver diseases such as primary sclerosing cholangitis and viral hepatitis.

JP2014167446 discloses measuring a soluble LR11 concentration in a blood-derived sample from a mammal to evaluate the severity and prognosis prediction of liver disease (except liver cancer) in the mammal. Mahler et al (2013; Clinica Chimica Acta 424: 267-273) disclose determining if PR3-ANCA is a biomarker that differentiates ulcerative colitis (UC) from Crohn's disease (CrD).

### Citation List

### Patent Literature

Patent Literature 1: JP 2017-122603 A
Patent Literature 2: JP 2014-167446 A

### Summary of Invention

### Technical Problem

In the past, diagnosis of ulcerative colitis has been made by a comprehensive judgment based on clinical symptoms, endoscopic observation, irrigoscopy, and biopsy histological test. However, a molecular biological diagnostic standard involving the use of ulcerative-colitis-specific biomarkers has not yet been discovered. While Patent Literature 1 describes a mapping method for diagnosis of ulcerative colitis, this method is not easily performed because of the need for intestinal flora sampling and genetic analysis thereof.

Concerning primary sclerosing cholangitis, also, a conventional diagnosis has been made based on a non-specific standard such as clinical symptoms, and a molecular biological diagnostic standard involving the use of primary sclerosing cholangitis-specific biomarkers has not yet been discovered. While the method described in Patent Literature 2 is a method for diagnosis of liver diseases including primary sclerosing cholangitis and viral hepatitis, this method does not distinguish primary sclerosing cholangitis from other liver diseases to perform specific diagnosis of primary sclerosing cholangitis.

Under the above circumstances, the present invention provides techniques associated with molecular biological diagnosis of ulcerative colitis and primary sclerosing cholangitis. Solution to Problem

The present inventors discovered that the concentration of an autoantibody to integrin αVβ6 and the concentration of an autoantibody to integrin αVβ3 were significantly high in the blood samples obtained from patients with ulcerative colitis and patients with primary sclerosing cholangitis. This has led to the completion of the inventions described below.

The present invention provides a method for testing ulcerative colitis comprising:
a step of detection comprising detecting, as an indicator of ulcerative colitis, an antibody immunologically reacting with full-length or a fragment of integrin αVβ6 and/or an antibody immunologically reacting with full-length or a fragment of integrin αVβ3 in a specimen; wherein the specimen is a blood sample, wherein;
a) the fragment of integrin αVβ6 is a dimer composed of an αV chain comprising Phe 31 to Val 992 of the amino acid sequence of the αV chain as shown in SEQ ID NO: 1 and a β6 chain comprising Gly 22 to Asn 707 of the amino acid sequence of the β6 chain as shown in SEQ ID NO: 2, and;
b) the fragment of integrin αVβ3 is a dimer composed of an αV chain comprising Phe 31 to Val 992 of the amino acid sequence of the αV chain as shown in SEQ ID NO: 1 and a β3 chain comprising Gly 27 to Asp 718 of the amino acid sequence of the β3 chain as shown in SEQ ID NO: 3.

The present invention further provides use of a test kit for testing ulcerative colitis on a blood sample wherein said test kit comprises full-length or a fragment of integrin αVβ6 and/or full-length or a fragment of integrin αVβ3, wherein;
a) the fragment of integrin αVβ6 is a dimer composed of an αV chain comprising Phe 31 to Val 992 of the amino acid sequence of the αV chain as shown in SEQ ID NO: 1 and a β6 chain comprising Gly 22 to Asn 707 of the amino acid sequence of the β6 chain as shown in SEQ ID NO: 2, and;
b) the fragment of integrin αVβ3 is a dimer composed of an αV chain comprising Phe 31 to Val 992 of the amino acid sequence of the αV chain as shown in SEQ ID NO: 1 and a β3 chain comprising Gly 27 to Asp 718 of the amino acid sequence of the β3 chain as shown in SEQ ID NO: 3.

In another aspect, the present invention provides a method for testing primary sclerosing cholangitis comprising:
a step of detection comprising detecting, as an indicator of primary sclerosing cholangitis, an antibody immunologically reacting with full-length or a fragment of integrin αVβ6 and/or an antibody immunologically reacting with full-length or a fragment of integrin αVβ3 in a specimen; wherein the specimen is a blood sample, wherein;
a) the fragment of integrin αVβ6 is a dimer composed of an αV chain comprising Phe 31 to Val 992 of the amino acid sequence of the αV chain as shown in SEQ ID NO: 1 and a β6 chain comprising Gly 22 to Asn 707 of the amino acid sequence of the β6 chain as shown in SEQ ID NO: 2, and;
b) the fragment of integrin αVβ3 is a dimer composed of an αV chain comprising Phe 31 to Val 992 of the amino acid sequence of the αV chain as shown in SEQ ID NO: 1 and a β3 chain comprising Gly 27 to Asp 718 of the amino acid sequence of the β3 chain as shown in SEQ ID NO: 3.

In a further aspect, the present invention use of a test kit for testing primary sclerosing cholangitis on a blood sample wherein said test kit comprises full-length or a fragment of integrin αVβ6 and/or full-length or a fragment of integrin αVβ3, wherein;
a) the fragment of integrin αVβ6 is a dimer composed of an αV chain comprising Phe 31 to Val 992 of the amino acid sequence of the αV chain as shown in SEQ ID NO: 1 and a β6 chain comprising Gly 22 to Asn 707 of the amino acid sequence of the β6 chain as shown in SEQ ID NO: 2, and;
b) the fragment of integrin αVβ3 is a dimer composed of an αV chain comprising Phe 31 to Val 992 of the amino acid sequence of the αV chain as shown in SEQ ID NO: 1 and a β3 chain comprising Gly 27 to Asp 718 of the amino acid sequence of the β3 chain as shown in SEQ ID NO: 3.

### Advantageous Effects of Invention

The method for testing ulcerative colitis according to the present invention and the test kit used therefor enable detection of ulcerative colitis with high sensitivity and specificity, and ulcerative colitis can be distinguished from Crohn's disease or other inflammatory bowel diseases and specifically detected.

The method for testing primary sclerosing cholangitis according to the present invention and the test kit used therefor enable detection of primary sclerosing cholangitis with high sensitivity and specificity, and primary sclerosing cholangitis can be distinguished from IgG4-related sclerosing cholangitis and other hepatobiliary diseases and specifically detected.

### Brief Description of the Drawings

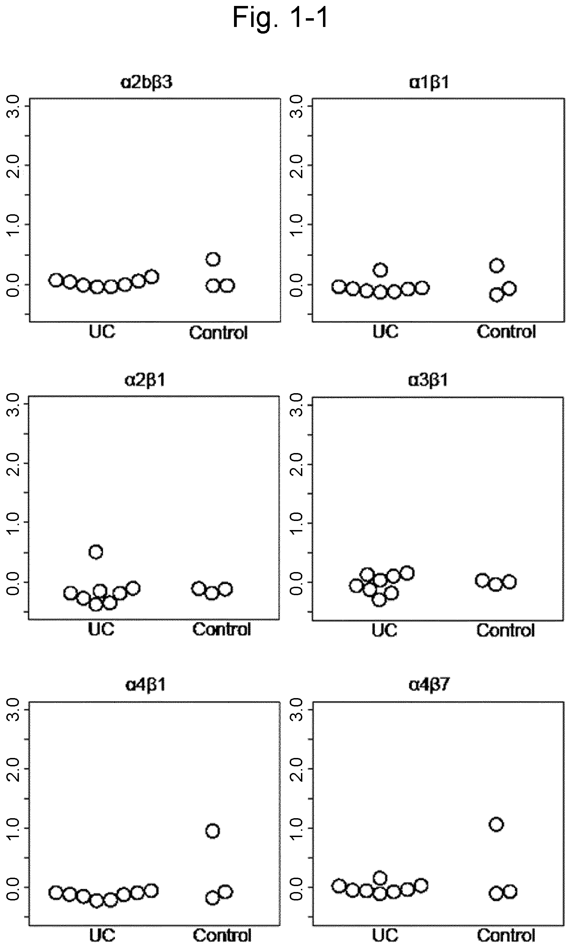

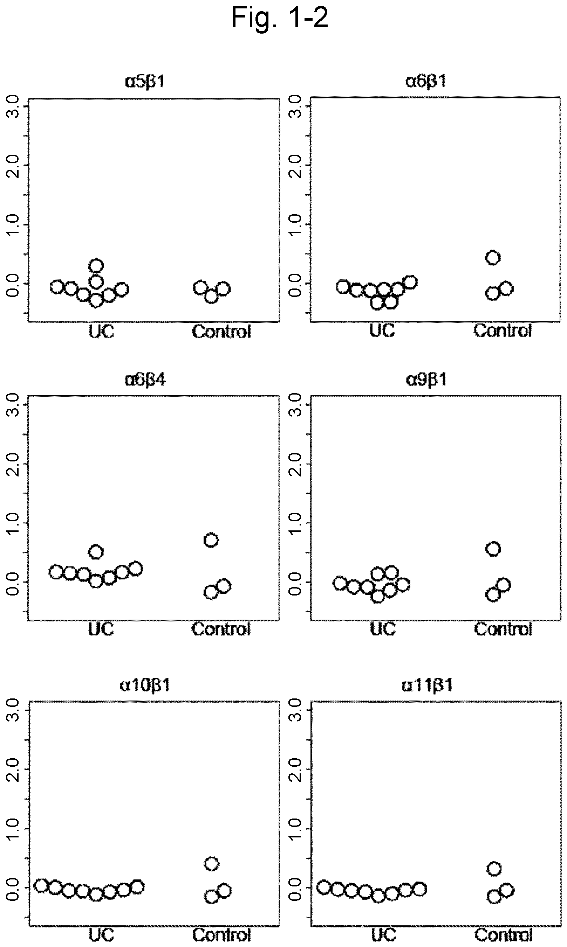

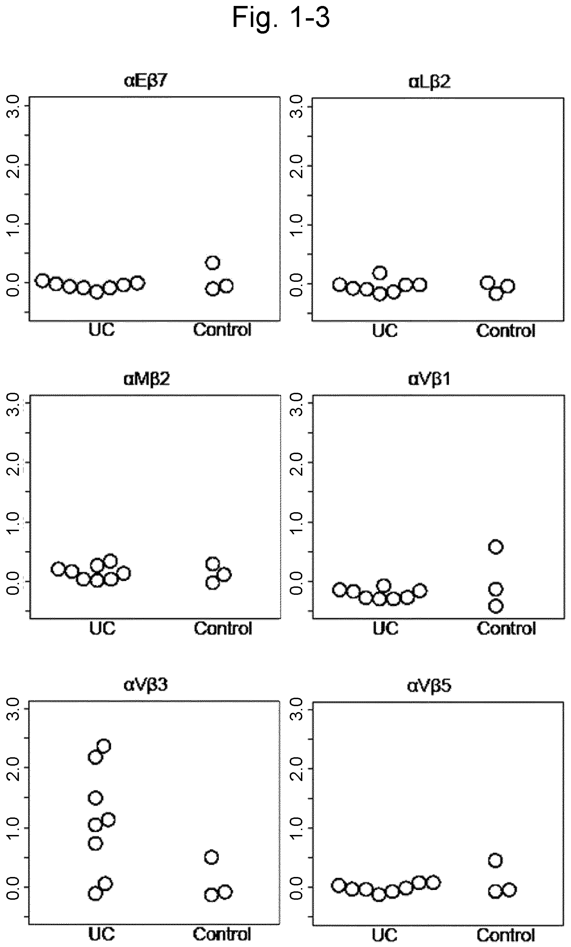

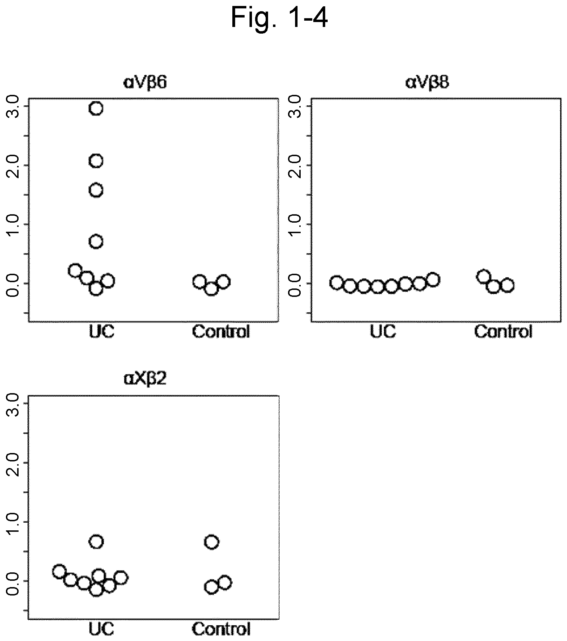

Fig. 1-1 shows concentration of the antibody to a plurality of human integrins comprising different combinations of α chain and β chain subunits in the serum samples of patients with ulcerative colitis (UC) and in the control serum samples (Control).
Fig. 1-2 shows concentration of the antibody to a plurality of human integrins comprising different combinations of α chain and β chain subunits in the serum samples of patients with ulcerative colitis (UC) and in the control serum samples (Control).
Fig. 1-3 shows concentration of the antibody to a plurality of human integrins comprising different combinations of α chain and β chain subunits in the serum samples of patients with ulcerative colitis (UC) and in the control serum samples (Control).
Fig. 1-4 shows concentration of the antibody to a plurality of human integrins comprising different combinations of α chain and β chain subunits in the serum samples of patients with ulcerative colitis (UC) and in the control serum samples (Control).
Fig. 2 shows concentration of the anti-human integrin αVβ6 antibody in the serum samples of patients with ulcerative colitis, in the serum samples of patients with Crohn's disease, and in the control serum samples.
Fig. 3 shows concentration of the anti-human integrin αVβ6 antibody in the serum samples of patients with primary sclerosing cholangitis, in the serum samples of patients with IgG4-related sclerosing cholangitis, and in the control serum samples.
Fig. 4 shows concentration of the anti-human integrin αVβ3 antibody in the serum samples of patients with ulcerative colitis, in the serum samples of patients with Crohn's disease, and in the control serum samples.
Fig. 5 shows concentration of the anti-human integrin αVβ3 antibody in the serum samples of patients with primary sclerosing cholangitis, in the serum samples of patients with IgG4-related sclerosing cholangitis, and in the control serum samples.
Fig. 6 shows concentration of the anti-human integrin αVβ6 antibody in the serum samples of patients with ulcerative colitis, in the serum samples of patients with Crohn's disease, and the serum samples of patients with other intestinal diseases.
Fig. 7 shows concentration of the anti-human integrin αVβ6 antibody in the serum samples of patients with ulcerative colitis, in the serum samples of patients with Crohn's disease, in the serum samples of patients with other intestinal diseases, in the serum samples of patients with collagen disease, and in the serum samples of healthy subjects.
Fig. 8A shows concentration of the anti-human integrin αVβ6 antibody IgG1 in the serum samples of patients with ulcerative colitis, in the serum samples of patients with Crohn's disease, and in the serum samples of patients with other intestinal diseases.
Fig. 8B shows concentration of the anti-human integrin αVβ6 antibody IgG2 in the serum samples of patients with ulcerative colitis, in the serum samples of patients with Crohn's disease, and in the serum samples of patients with other intestinal diseases.
Fig. 8C shows concentration of the anti-human integrin αVβ6 antibody IgG3 in the serum samples of patients with ulcerative colitis, in the serum samples of patients with Crohn's disease, and in the serum samples of patients with other intestinal diseases.
Fig. 8D shows concentration of the anti-human integrin αVβ6 antibody IgG4 in the serum samples of patients with ulcerative colitis, in the serum samples of patients with Crohn's disease, and in the serum samples of patients with other intestinal diseases.
Fig. 9A shows concentration of the anti-human integrin αVβ6 antibody IgA in the serum samples of patients with ulcerative colitis, in the serum samples of patients with Crohn's disease, and in the serum samples of patients with other intestinal diseases.
Fig. 9B shows concentration of the anti-human integrin αVβ6 antibody IgM in the serum samples of patients with ulcerative colitis, in the serum samples of patients with Crohn's disease, and in the serum samples of patients with other intestinal diseases.
Fig. 9C shows concentration of the anti-human integrin αVβ6 antibody IgE in the serum samples of patients with ulcerative colitis, in the serum samples of patients with Crohn's disease, and in the serum samples of patients with other intestinal diseases.
Fig. 10A shows the partial Mayo scores of a patient with ulcerative colitis at each point during a period from November, 2017 to November, 2018 (the left vertical axis) and the antibody titers of the anti-human integrin αVβ6 autoantibody (IgG) (the right vertical axis).
Fig. 10B shows the partial Mayo scores of another patient with ulcerative colitis at each point during a period from November, 2017 to November, 2018 (the left vertical axis) and the antibody titers of the anti-human integrin αVβ6 autoantibody (IgG) (the right vertical axis).
Fig. 11 shows concentration of the anti-human integrin αVβ3 antibody in the serum samples of patients with ulcerative colitis, in the serum samples of patients with Crohn's disease, and in the serum samples of patients with other intestinal diseases.
Fig. 12 shows concentration of the anti-human integrin αVβ3 antibody in the serum samples of patients with ulcerative colitis, in the serum samples of patients with Crohn's disease, in the serum samples of patients with other intestinal diseases, in the serum samples of patients with collagen disease, and in the serum samples of healthy subjects.
Fig. 13A shows concentration of the anti-human integrin αVβ3 antibody IgG1 in the serum samples of patients with ulcerative colitis, in the serum samples of patients with Crohn's disease, and in the serum samples of patients with other intestinal diseases.
Fig. 13B shows concentration of the anti-human integrin αVβ3 antibody IgG2 in the serum samples of patients with ulcerative colitis, in the serum samples of patients with Crohn's disease, and in the serum samples of patients with other intestinal diseases.
Fig. 13C shows concentration of the anti-human integrin αVβ3 antibody IgG3 in the serum samples of patients with ulcerative colitis, in the serum samples of patients with Crohn's disease, and in the serum samples of patients with other intestinal diseases.
Fig. 13D shows concentration of the anti-human integrin αVβ3 antibody IgG4 in the serum samples of patients with ulcerative colitis, in the serum samples of patients with Crohn's disease, and in the serum samples of patients with other intestinal diseases.
Fig. 14A shows concentration of the anti-human integrin αVβ3 antibody IgA in the serum samples of patients with ulcerative colitis, in the serum samples of patients with Crohn's disease, and in the serum samples of patients with other intestinal diseases.
Fig. 14B shows concentration of the anti-human integrin αVβ3 antibody IgM in the serum samples of patients with ulcerative colitis, in the serum samples of patients with Crohn's disease, and in the serum samples of patients with other intestinal diseases.
Fig. 14C shows concentration of the anti-human integrin αVβ3 antibody IgE in the serum samples of patients with ulcerative colitis, in the serum samples of patients with Crohn's disease, and in the serum samples of patients with other intestinal diseases.

### Description

### <1. Specimens>

A test subject subjected to the test method according to the present invention is not particularly limited. A test subject may be a human or other non-human mammalian animal, with a human being preferable.

Examples of specimens used in the test method described herein include body fluids sampled from a test subject. Specific examples thereof include blood samples, such as serum, plasma, and whole blood samples, and body fluid samples other than blood samples, such as saliva, spinal fluid, and urine samples. In addition to body fluids, tissue samples obtained from a test subject, such as tissue samples obtained from the disease site of ulcerative colitis to be tested (e.g., the colon and the rectum) or tissue samples obtained from the disease site of primary sclerosing cholangitis (e.g., the liver) can be used as specimens. The specimens are isolated from the test subject and used in that state in the test method as described herein. The specimen used in the present invention is a blood sample.

### <2. Integrin αVβ6>

A natural integrin is a protein comprising heterodimeric molecules composed of 2 subunit chains; an α chain and a β chain. α1 to α11, αV, αX, αM, αL, αD, αE, and αIIb chains and β1 to β8 chains are known, and there are a plurality of isoforms composed of different combinations of subunit chains.

Integrin is present on the epithelial cell surface and it binds to an extracellular matrix protein on the binding-tissue surface, such as laminin or fibronectin.

Integrin αVβ6 comprises heterodimeric molecules composed of an αV chain and a β6 chain. While integrin αVβ6 is not substantially expressed in normal tissue, it is expressed on the epithelial cell surface upon, for example, inflammatory stimulation.

In the present description, an origin of full-length or a fragment of integrin αVβ6 that immunologically binds to an autoantibody to be detected is not particularly limited. An origin is preferably of the same species as the test subject, with a human being particularly preferable. Nucleotide sequence information of the gene encoding the αV chain and the β6 chain of the integrin of a mammalian species such as a human and amino acid sequence information of the chains can be obtained from a known database (e.g., GenBank). In particular, the amino acid sequence of a preproprotein of the human integrin αV chain isoform 1 is registered under GenBank Accession Number NP_002201.2, and the sequence thereof is shown in SEQ ID NO: 1. The amino acid sequence of a human integrin β6 chain precursor is registered under GenBank Accession Number NP_000879.2, and the sequence thereof is shown in SEQ ID NO: 2. Amino acid sequence information of the integrin αV and β6 chains of various mammalian animals other than humans can also be obtained from a known database (e.g., GenBank). The integrin αVβ6 may be composed of the αV chain and the β6 chain comprising the amino acid sequences resulting from post-translational modification of either or both the amino acid sequences of the αV chain and the β6 chain registered in the database. For example, a partial sequence of amino acids 1 to 30 of the amino acid sequence as shown in SEQ ID NO: 1 is a signal peptide sequence, and the amino acid sequence of a mature polypeptide in the human integrin αV chain includes a partial sequence of amino acids 31 to 1048 of the amino acid sequence as shown in SEQ ID NO: 1. Also, a partial sequence of amino acids 1 to 21 of the amino acid sequence as shown in SEQ ID NO: 2 is a signal peptide sequence, and the amino acid sequence of a mature polypeptide in the human integrin β6 chain includes a partial sequence of amino acids 22 to 788 of the amino acid sequence as shown in SEQ ID NO: 2.

In the present description, in addition, integrin αVβ6 is not limited to a natural integrin comprising a mature or immature amino acid sequence, unless otherwise specified. It may be a variant equivalent to the natural integrin αVβ6.

Integrin αVβ6 is not limited to a natural or variant integrin αVβ6 comprising the full length α chain and the full-length β chain, each comprising a mature or immature amino acid sequence (i.e., full-length integrin αVβ6), and it may be a fragment of integrin αVβ6.

An autoantibody to be detected is not limited to an antibody that immunologically binds to full-length or a fragment of integrin αVβ6 in which each of the chains consists only of the mature or immature amino acid sequence. Such autoantibody may be an antibody that immunologically binds to full-length or a fragment of integrin αVβ6 in which each of the chains comprises additional peptides added to the mature or immature amino acid sequence (in particular, full-length or a fragment of integrin αVβ6 in which each of the chains comprises an additional peptide added to the C terminus of the mature or immature amino acid sequence).

An example of an integrin αVβ6 fragment is a fragment in which at least either one of the αV chain and the β6 chain constituting the integrin dimer is shorter than the corresponding chain in a mature or immature nartural integrin or a variant thereof. A specific example of an integrin αVβ6 fragment is a dimer composed of an αV chain comprising a partial sequence of Phe 31 to Val 992 of the amino acid sequence of the αV chain as shown in SEQ ID NO: 1 and/or a β6 chain comprising a partial sequence of Gly 22 to Asn 707 of the amino acid sequence of the β6 chain as shown in SEQ ID NO: 2. It is preferable that an integrin αVβ6 fragment form a dimer, and it is more preferable that it have activity of binding to an extracellular matrix protein, such as laminin or fibronectin. Whether or not an integrin αVβ6 fragment has activity of binding to an extracellular matrix protein can be examined via ELISA or other means.

Whether or not the full-length or a fragment of integrin αVβ6 forms a dimer can be determined in the manner described below. That is, the full-length or a fragment of integrin αVβ6 is subjected to SDS-PAGE in the absence of 2-mercaptoethanol and a band equivalent to its dimeric molecular weight is detected. Simultaneously, the full-length or a fragment of integrin αVβ6 is subjected to SDS-PAGE in the presence of 2-mercaptoethanol and a band equivalent to its dimeric molecular weight is quenched. Thus, whether or not the full-length or a fragment of integrin αVβ6 forms a dimer can be determined.

An example of commercially available integrin αVβ6 is recombinant human integrin αVβ6 (R&D Systems, Minnesota, U.S.A., Product Number: 3817-AV). This recombinant human integrin αVβ6 is a dimer composed of: an αV chain comprising a partial sequence of Phe 31 to Val 992 of the amino acid sequence of the αV chain as shown in SEQ ID NO: 1, and a linker sequence and an acidic tail sequence added to the C terminus thereof; and a β6 chain comprising a partial sequence of Gly 22 to Asn 707 of the amino acid sequence of the β6 chain as shown in SEQ ID NO: 2, and a linker sequence and a basic tail sequence added to the C terminus thereof.

A more specific example of an αV chain constituting full-length or a fragment of integrin αVβ6 is a polypeptide selected from the group consisting of the following:
(I) a polypeptide comprising the amino acid sequence as shown in SEQ ID NO: 1 or a partial sequence of Phe 31 to Thr 1048 of the amino acid sequence as shown in SEQ ID NO: 1;
(II) a polypeptide comprising a partial sequence of the amino acid sequence as shown in SEQ ID NO: 1 and functionally equivalent to the polypeptide (I);
(III) a polypeptide comprising an amino acid sequence having 85% or higher sequence identity to the amino acid sequence as shown in SEQ ID NO: 1 or a partial sequence thereof and functionally equivalent to the polypeptide (I); and
(IV) a polypeptide comprising an amino acid sequence derived from the amino acid sequence as shown in SEQ ID NO: 1 or a partial sequence thereof by substitution, deletion, and/or addition of 1 or a plurality of amino acids and functionally equivalent to the polypeptide (I).

The polypeptides (I) to (IV) above may each comprise an amino acid sequence derived from each of the amino acid sequences or the partial sequences as defined in (I) to (IV) by addition of an additional amino acid sequence to at least one of the N terminus or the C terminus (preferably to the C terminus).

In (II), (III), and (IV), a polypeptide functionally equivalent to the polypeptide (I) can form a dimer with an integrin β6 chain (particularly preferably, a polypeptide chain consisting of the amino acid sequence as shown in SEQ ID NO: 2, a polypeptide chain consisting of a partial sequence of Gly 22 to Cys 788 of the amino acid sequence as shown in SEQ ID NO: 2, or a polypeptide chain consisting of a partial sequence of Gly 22 to Asn 707 of the amino acid sequence as shown in SEQ ID NO: 2). In addition, the resulting dimer has an ability of binding to an extracellular matrix protein, such as laminin or fibronectin, to which natural integrin αVβ6 or commercially available integrin αVβ6 can bind.

An example of the partial sequence as defined in (II) is a partial sequence of Phe 31 to Val 992 of the amino acid sequence as shown in SEQ ID NO: 1. Examples of the partial sequences as defined in (III) and (IV) include a partial sequence of Phe 31 to Thr 1048 of the amino acid sequence as shown in SEQ ID NO: 1 and a partial sequence of Phe 31 to Val 992 of the amino acid sequence as shown in SEQ ID NO: 1.

Sequence identity as defined in (III) is preferably 90% or higher, more preferably 95% or higher, further preferably 96% or higher, particularly preferably 97% or higher, and most preferably 98% or higher or 99% or higher.

In (IV), the number represented by the term "1 or a plurality of" is, for example, 1 to 100, preferably 1 to 50, more preferably 1 to 30, more preferably 1 or 20, more preferably 1 to 15, more preferably 1 to 10, more preferably 1 to 5, more preferably 1 to 4, more preferably 1 to 3, more preferably 1 or 2, and most preferably 1.

A more specific example of a β6 chain constituting full-length or a fragment of integrin αVβ6 is a polypeptide selected from the group consisting of the following:
(V) a polypeptide comprising the amino acid sequence as shown in SEQ ID NO: 2 or a partial sequence of Gly 22 to Cys 788 of the amino acid sequence as shown in SEQ ID NO: 2;
(VI) a polypeptide comprising a partial sequence of the amino acid sequence as shown in SEQ ID NO: 2 and functionally equivalent to the polypeptide (V);
(VII) a polypeptide comprising an amino acid sequence having 85% or higher sequence identity to the amino acid sequence as shown in SEQ ID NO: 2 or a partial sequence thereof and functionally equivalent to the polypeptide (V); and
(VIII) a polypeptide comprising an amino acid sequence derived from the amino acid sequence as shown in SEQ ID NO: 2 or a partial sequence thereof by substitution, deletion, and/or addition of 1 or a plurality of amino acids and functionally equivalent to the polypeptide (V).

The polypeptides (V) to (VIII) above may each comprise an amino acid sequence derived from each of the amino acid sequences or the partial sequences as defined in (V) to (VIII) by addition of an additional amino acid sequence to at least one of the N terminus or the C terminus (preferably to the C terminus).

In (VI), (VII), and (VIII), a polypeptide functionally equivalent to the polypeptide (V) can form a dimer with an integrin αV chain (particularly preferably, a polypeptide chain consisting of the amino acid sequence as shown in SEQ ID NO: 1, a polypeptide chain consisting of a partial sequence of Phe 31 to Thr 1048 of the amino acid sequence as shown in SEQ ID NO: 1, or a polypeptide chain consisting of a partial sequence of Phe 31 to Val 992 of the amino acid sequence as shown in SEQ ID NO: 1). In addition, the resulting dimer has an ability of binding to an extracellular matrix protein, such as laminin or fibronectin, to which natural integrin αVβ6 or commercially available integrin αVβ6 can bind.

An example of the partial sequence as defined in (VI) is a partial sequence of Gly 22 to Asn 707 of the amino acid sequence as shown in SEQ ID NO: 2. Examples of the partial sequences as defined in (VII) and (VIII) include a partial sequence of Gly 22 to Cys 788 of the amino acid sequence as shown in SEQ ID NO: 2 and a partial sequence of Gly 22 to Asn 707 of the amino acid sequence as shown in SEQ ID NO: 2.

Sequence identity as defined in (VII) is preferably 90% or higher, more preferably 95% or higher, further preferably 96% or higher, particularly preferably 97% or higher, and most preferably 98% or higher or 99% or higher.

In (VIII), the number represented by the term "1 or a plurality of" is, for example, 1 to 100, preferably 1 to 50, more preferably 1 to 30, more preferably 1 or 20, more preferably 1 to 15, more preferably 1 to 10, more preferably 1 to 5, more preferably 1 to 4, more preferably 1 to 3, more preferably 1 or 2, and most preferably 1.

In (III) and (VII) above and (XI) described below, amino acid sequence identity can be determined, for example, in accordance with a method well known in the art or with the use of sequence analysis software. Examples of sequence analysis software include the blastp program of the BLAST algorithm and the fasta program of the FASTA algorithm.

### <3. Integrin αVβ3>

Integrin αVβ3 comprises heterodimeric molecules composed of an αV chain and a β3 chain.

In the present description, an origin of full-length or a fragment of integrin αVβ3 that immunologically binds to an autoantibody to be detected is not particularly limited. An origin is preferably of the same species as the test subject, with a human being particularly preferable. Nucleotide sequence information of the gene encoding the αV chain and the β3 chain of the integrin of a mammalian species such as a human and amino acid sequence information of the chains can be obtained from a known database (e.g., GenBank).

Information concerning the αV chain is as described above and description thereof is thus omitted herein.

The amino acid sequence of a human integrin β3 chain precursor is registered under GenBank Accession Number AAA52589.1, and the sequence thereof is shown in SEQ ID NO: 3. Amino acid sequence information of the integrin β3 chain of various mammalian animals other than humans can also be obtained from a known database (e.g., GenBank).

The integrin αVβ3 may be composed of the αV chain and the β3 chain comprising the amino acid sequences resulting from post-translational modification of either or both the amino acid sequences of the αV chain and the β3 chain registered in the database. For example, the amino acid sequence of a mature polypeptide in the human integrin αV chain includes a partial sequence of amino acids 31 to 1048 of the amino acid sequence as shown in SEQ ID NO: 1. Also, a partial sequence of amino acids 1 to 26 of the amino acid sequence as shown in SEQ ID NO: 3 is a signal peptide sequence, and the amino acid sequence of a mature polypeptide in the human integrin β3 chain includes a partial sequence of amino acids 27 to 788 of the amino acid sequence as shown in SEQ ID NO: 3.

In the present description, in addition, integrin αVβ3 is not limited to a natural integrin comprising a mature or immature amino acid sequence, unless otherwise specified. It may be a variant equivalent to the natural integrin αVβ3.

Integrin αVβ3 is not limited to a natural or variant integrin αVβ3 comprising the full length α chain and the full-length β chain, each comprising a mature or immature amino acid sequence (i.e., full-length integrin αVβ3), and it may be a fragment of integrin αVβ3.

An autoantibody to be detected is not limited to an antibody that immunologically binds to full-length or a fragment of integrin αVβ3 in which each of the chains consists only of the mature or immature amino acid sequence. Such autoantibody may be an antibody that immunologically binds to full-length or a fragment of integrin αVβ3 in which each of the chains comprises additional peptides added to the mature or immature amino acid sequence (in particular, full-length or a fragment of integrin αVβ3 in which each of the chains comprises an additional peptide added to the C terminus of the mature or immature amino acid sequence).

An example of an integrin αVβ3 fragment is a fragment in which at least either one of the αV chain and the β3 chain constituting the integrin dimer is shorter than the corresponding chain in a mature or immature nartural integrin or a variant thereof. A specific example of an integrin αVβ3 fragment is a dimer composed of an αV chain comprising a partial sequence of Phe 31 to Val 992 of the amino acid sequence of the αV chain as shown in SEQ ID NO: 1 and/or a β3 chain comprising a partial sequence of Gly 27 to Asp 718 of the amino acid sequence of the β3 chain as shown in SEQ ID NO: 3. It is preferable that an integrin αVβ3 fragment form a dimer, and it is more preferable that it have activity of binding to an extracellular matrix protein, such as laminin or fibronectin. Whether or not an integrin αVβ3 fragment has activity of binding to an extracellular matrix protein can be examined via ELISA or other means.

Whether or not the full-length or a fragment of integrin αVβ3 forms a dimer can be determined in the manner described below. That is, the full-length or a fragment of integrin αVβ3 is subjected to SDS-PAGE in the absence of 2-mercaptoethanol and a band equivalent to its dimeric molecular weight is detected. Simultaneously, the full-length or a fragment of integrin αVβ3 is subjected to SDS-PAGE in the presence of 2-mercaptoethanol and a band equivalent to its dimeric molecular weight is quenched. Thus, whether or not the full-length or a fragment of integrin αVβ3 forms a dimer can be determined.

An example of commercially available integrin αVβ3 is recombinant human integrin αVβ3 (R&D Systems, Minnesota, U.S.A., Product Number: 3050-AV). This recombinant human integrin αVβ3 is a dimer composed of: an αV chain comprising a partial sequence of Phe 31 to Val 992 of the amino acid sequence of the αV chain as shown in SEQ ID NO: 1, and a linker sequence and an acidic tail sequence added to the C terminus thereof; and a β3 chain comprising a partial sequence of Gly 27 to Asp 718 of the amino acid sequence of the β3 chain as shown in SEQ ID NO: 3, and a linker sequence and a basic tail sequence added to the C terminus thereof.

A more specific example of an αV chain constituting full-length or a fragment of integrin αVβ3 is a polypeptide selected from among (I) to (IV) above.

A more specific example of a β3 chain constituting full-length or a fragment of integrin αVβ3 is a polypeptide selected from the group consisting of the following:
(IX) a polypeptide comprising the amino acid sequence as shown in SEQ ID NO: 3, a partial sequence of Gly 27 to Asp 718 of the amino acid sequence as shown in SEQ ID NO: 3, or a partial sequence of Gly 27 to Thr 788 of the amino acid sequence as shown in SEQ ID NO: 3;
(X) a polypeptide comprising a partial sequence of the amino acid sequence as shown in SEQ ID NO: 3 and functionally equivalent to the polypeptide (IX);
(XI) a polypeptide comprising an amino acid sequence having 85% or higher sequence identity to the amino acid sequence as shown in SEQ ID NO: 3 or a partial sequence thereof and functionally equivalent to the polypeptide (IX); and
(XII) a polypeptide comprising an amino acid sequence derived from the amino acid sequence as shown in SEQ ID NO: 3 or a partial sequence thereof by substitution, deletion, and/or addition of 1 or a plurality of amino acids and functionally equivalent to the polypeptide (IX).

The polypeptides (IX) to (XIII) above may each comprise an amino acid sequence derived from each of the amino acid sequences or the partial sequences as defined in (IX) to (XIII) by addition of an additional amino acid sequence to at least one of the N terminus or the C terminus (preferably to the C terminus).

In (X) to (XII), a polypeptide functionally equivalent to the polypeptide (IX) can form a dimer with an integrin αV chain (particularly preferably, a polypeptide chain consisting of the amino acid sequence as shown in SEQ ID NO: 1, a polypeptide chain consisting of a partial sequence of Phe 31 to Thr 1048 of the amino acid sequence as shown in SEQ ID NO: 1, or a polypeptide chain consisting of a partial sequence of Phe 31 to Val 992 of the amino acid sequence as shown in SEQ ID NO: 1). In addition, the resulting dimer has an ability of binding to an extracellular matrix protein, such as laminin or fibronectin, to which natural integrin αVβ3 or commercially available integrin αVβ3 can bind.

An example of the partial sequence as defined in (X) is a partial sequence of Gly 27 to Asp 718 of the amino acid sequence as shown in SEQ ID NO: 3. Examples of the partial sequences as defined in (XI) and (XII) include a partial sequence of Gly 27 to Asp 718 of the amino acid sequence as shown in SEQ ID NO: 3 and a partial sequence of Gly 27 to Thr 788 of the amino acid sequence as shown in SEQ ID NO: 3.

Sequence identity as defined in (XI) is preferably 90% or higher, more preferably 95% or higher, further preferably 96% or higher, particularly preferably 97% or higher, and most preferably 98% or higher or 99% or higher.

In (XII), the number represented by the term "1 or a plurality of" is, for example, 1 to 100, preferably 1 to 50, more preferably 1 to 30, more preferably 1 or 20, more preferably 1 to 15, more preferably 1 to 10, more preferably 1 to 5, more preferably 1 to 4, more preferably 1 to 3, more preferably 1 or 2, and most preferably 1.

### <4. Method for testing ulcerative colitis or primary sclerosing cholangitis>

The first test method according to the present description relates to a method for testing ulcerative colitis comprising:
a step of detection comprising detecting an antibody immunologically reacting with full-length or a fragment of integrin αVβ6 and/or an antibody immunologically reacting with full-length or a fragment of integrin αVβ3 in a specimen as an indicator of ulcerative colitis.

The second test method according to the present description relates to a method for testing primary sclerosing cholangitis comprising:
a step of detection comprising detecting an antibody immunologically reacting with full-length or a fragment of integrin αVβ6 and/or an antibody immunologically reacting with full-length or a fragment of integrin αVβ3 in a specimen as an indicator of primary sclerosing cholangitis.

Hereafter, "full-length or a fragment of integrin αVβ6" is referred to as "integrin αVβ6," and "full-length or a fragment of integrin αVβ3" is referred to as "integrin αVβ3."

The first test method according to the present description was completed based on remarkable findings. That is, an antibody (autoantibody) to integrin αVβ6 and an antibody (autoantibody) to integrin αVβ3 in the specimen obtained from a test subject would serve as indicators of ulcerative colitis.

The second test method according to the present description was completed based on remarkable findings. That is, an antibody (autoantibody) to integrin αVβ6 and an antibody (autoantibody) to integrin αVβ3 in the specimen obtained from a test subject would serve as indicators of primary sclerosing cholangitis.

The step of detection of the test method according to the present invention comprises detecting an antibody immunologically reacting with integrin αVβ6 and/or an antibody immunologically reacting with integrin αVβ3 in a specimen, wherein the specimen is a blood sample. In the present invention, the term "detection" is a concept that includes examination of the presence or absence of an antibody immunologically reacting with integrin αVβ6 and/or an antibody immunologically reacting with integrin αVβ3 in a specimen and measurement of the amount of the antibody in the specimen; i.e., quantification.

The step of detection of the test method according to the present invention may be any method that can quantitatively or qualitatively detect the antibody in the specimen, and a specific embodiment is not particularly limited. For example, an immunological method can be adopted. Examples thereof include enzyme-linked immunosorbent assay (ELISA), immunoprecipitation assay (IPP), immunoblotting (IB), latex agglutination assay, immunochromatography, indirect fluorescent-antibody assay (IF), and radioimmunoassay (RIA). ELISA is particularly preferable, since it can process a large number of specimens. According to ELISA, specifically, antigens to the antibody, i.e., integrin αVβ6 and/or integrin αVβ3 as described in detail above, are immobilized on a solid phase, the specimens are brought into contact with the antigens immobilized on the solid phase, and an immune complex of the antigen to the antibody that may be contained in the specimen is detected with the use of an enzyme-labeled detection antibody or the like. Thus, the antibody of interest can be detected. When performing ELISA, a method for suppressing a non-specific reaction caused by ELISA, a label substance or an assay apparatus that can be used for detection, and the like are not particularly limited. A solid phase on which an antigen is to be immobilized can be of any form, such as a plate, bead, or tube.

According to an immunological technique such as ELISA, a step of bringing a specimen that may contain the antibody into contact with the antigen and, according to needs, steps of washing, labeling and/or detecting can be performed in an adequate buffer. A preferable buffer contains one or more types of metal ions selected from among calcium ion, magnesium ion, manganese ion, sodium ion, and lithium ion. Such metal ion is preferably a divalent metal ion, such as calcium ion, magnesium ion, or manganese ion. Particularly preferable metal ion is one or two selected from among calcium ion, magnesium ion, and manganese ion. While the concentration of the metal ion in the buffer is not particularly limited, for example, such concentration may be 0.02 mM to 200 mM in total, it is preferably 0.2 mM to 20 mM, and it is particularly preferably 0.4 mM to 10 mM. A buffer containing such metal ions is preferable because detection sensitivity is enhanced in such buffer.

In the step of detection, the amount of an antibody immunologically reacting with integrin αVβ and/or the amount of an antibody immunologically reacting with integrin αVβ3 can be determined as the amount of a labeled detection antibody that had bound to the immune complex of the antibody and the antigen. Alternatively, a calibration curve may be prepared with the aid of a positive standard sample solution containing the antibody at a known concentration, and the amount of the antibody in the test sample can be calculated based on the measured amount of the label using the calibration curve.

In the step of detection, the presence or absence of an antibody immunologically reacting with integrin αVβ6 and/or an antibody immunologically reacting with integrin αVβ3 can be determined (i.e., determination of positivity/negativity) by assaying the antibody in the specimen obtained from a test subject (e.g., a human suspected of having ulcerative colitis or primary sclerosing cholangitis), assaying the antibody in the specimen obtained from a control subject without ulcerative colitis or primary sclerosing cholangitis (e.g., a healthy subject), and comparing the assay results. The antibody in the specimen obtained from a control subject without ulcerative colitis or primary sclerosing cholangitis may be assayed at the time of the test or it may be assayed in advance. When the amount of the antibody assayed in the specimen obtained from the test subject is significantly larger than the amount of the antibody assayed in the specimen obtained from the control subject without ulcerative colitis or primary sclerosing cholangitis, the specimen obtained from the test subject can be determined to be antibody-positive.

Isotypes of an antibody immunologically reacting with integrin αVβ6 and/or an antibody immunologically reacting with integrin αVβ3 to be detected in the step of detection are not particularly limited. IgG, IgA, IgM, IgE or other isotypes may be detected, with IgG or IgA being particularly preferable. As the antibody immunologically reacting with integrin αVβ6, it is particularly preferable that IgG or IgA be detected. As the antibody immunologically reacting with integrin αVβ3, it is particularly preferable that IgG be detected. While IgG antibody subclasses are not particularly limited, for example, IgG1, IgG2, IgG3 or IgG4 can be detected. As IgG that immunologically reacts with integrin αVβ6, it is more preferable that IgG1, IgG2 or IgG4 be detected, and it is the most preferable that IgG1 be detected. As IgG that immunologically reacts with integrin αVβ3, it is more preferable that IgG1, IgG2 or IgG3 be detected, and it is the most preferable that IgG1 be detected.

### <5. Test kit or test reagent of ulcerative colitis or primary sclerosing cholangitis>

The first test kit or test reagent according to the present description relates to a test kit or test reagent for use in testing ulcerative colitis, which comprises full-length or a fragment of integrin αVβ6 and/or full-length or a fragment of integrin αVβ3.

The second test kit or test reagent according to the present description relates to a test kit or test reagent for use in testing primary sclerosing cholangitis, which comprises full-length or a fragment of integrin αVβ6 and/or full-length or a fragment of integrin αVβ3.

Hereafter, "full-length or a fragment of integrin αVβ6" is referred to as "integrin αVβ6," and "full-length or a fragment of integrin αVβ3" is referred to as "integrin αVβ3."

The test kit or test reagent according to the present description can be used in the test method according to the present invention.

The test kit according to the present description may comprise reagents for immunological assays, such as a buffer, at least one metal salt selected from among calcium salt, magnesium salt, manganese salt, sodium salt, and lithium salt, and the like, according to need.

The test reagent according to the present description can be a liquid or solid composition comprising integrin αVβ6 and/or integrin αVβ3 and a carrier, such as a solvent or excipient, acceptable for formuration.

In the test kit or test reagent according to the present description, the form of integrin αVβ6 and/or integrin αVβ3 is not particularly limited, and integrin αVβ6 and/or integrin αVβ3 may be in the form of a solution, in a dried state, or immobilized on a solid phase. When integrin αVβ6 and/or integrin αVβ3 is in a dried state, the test kit or test reagent according to the present description may be supplemented with a buffer or solvent so as to prepare a solution before use.

The test kit or test reagent comprising integrin αVβ6 and/or integrin αVβ3 immobilized on a solid phase can be used for testing ulcerative colitis or primary sclerosing cholangitis via ELISA. The solid phase may be of any form, such as a plate, bead, or tube. The test kit for testing ulcerative colitis or primary sclerosing cholangitis via ELISA can contain, in addition to the solid phase, a positive standard sample solution, a negative standard sample solution, a blocking solution, a wash solution, a specimen diluent, a detection antibody, a substrate solution, and the like. The detection antibody may be labeled with a detectable label, such as an enzyme. The positive standard sample solution may be a diluted serum obtained from a patient with ulcerative colitis or primary sclerosing cholangitis containing the autoantibody to integrin αVβ6 and/or integrin αVβ3, or may be a solution supplemented with an anti-integrin αVβ6 antibody and/or an anti-integrin αVβ3 antibody. The isotype of the antibody to be added to the solution is preferably the same as the isotype of the autoantibody to be detected, such as the IgG antibody, the IgA antibody, the IgM antibody, or the IgE antibody. The negative standard sample solution may be, for example, a diluted serum obtained from a person without ulcerative colitis or primary sclerosing cholangitis, a serum obtained from a person with other intestinal diseases that are medically distinguished from ulcerative colitis, a serum obtained from a person with other diseases of the hepatobiliary system that are medically distinguished from primary sclerosing cholangitis, a serum obtained from a healthy subject (the control serum sample), or a diluted solution of such serum samples.

### <6. Method for evaluating therapeutic effects on ulcerative colitis or primary sclerosing cholangitis>

The first method for evaluating therapeutic effects according to the present description relates to a method for evaluating effects of treatment on ulcerative colitis comprising:
a step of detection comprising detecting an antibody immunologically reacting with full-length or a fragment of integrin αVβ6 and/or an antibody immunologically reacting with full-length or a fragment of integrin αVβ3 in a specimen obtained from a test subject subjected to treatment of ulcerative colitis.

The second method for evaluating therapeutic effects according to the present description relates to a method for evaluating effects of treatment on primary sclerosing cholangitis comprising:
a step of detection comprising detecting an antibody immunologically reacting with full-length or a fragment of integrin αVβ6 and/or an antibody immunologically reacting with full-length or a fragment of integrin αVβ3 in a specimen obtained from a test subject subjected to treatment of primary sclerosing cholangitis.

Hereafter, "full-length or a fragment of integrin αVβ6" is referred to as "integrin αVβ6," and "full-length or a fragment of integrin αVβ3" is referred to as "integrin αVβ3."

In the test method according to the present invention, as described above, an antibody immunologically reacting with integrin αVβ6 and an antibody immunologically reacting with integrin αVβ3 in the specimen are useful as indicators of ulcerative colitis or primary sclerosing cholangitis. When ulcerative colitis or primary sclerosing cholangitis is cured because of effective treatment, accordingly, the amount of the antibody in the specimen obtained from the test subject subjected to treatment of ulcerative colitis or primary sclerosing cholangitis would be decreased. When effective therapeutic effects are not exerted, such amount would not be changed or increased from the amount before the initiation of treatment. By detecting the antibody in the specimen obtained from the test subject subjected to treatment of ulcerative colitis or primary sclerosing cholangitis, accordingly, therapeutic effects can be evaluated.

Examples of test subjects include humans and non-human animals subjected to treatment of ulcerative colitis or primary sclerosing cholangitis.

Concerning the method for evaluating therapeutic effects according to the present description, specific embodiments of the specimen, the integrin αVβ6, the integrin αVβ3, the antibody, the step of detection, and the like are as described above with regard to the test method according to the present invention.

The results of quantification of the antibody in the specimen obtained from the test subject subjected to treatment of ulcerative colitis or primary sclerosing cholangitis obtained in the step of detection in the method for evaluating therapeutic effects according to the present description are compared with, for example, the results of quantification of the antibody in the specimen obtained from the same test subject before the initiation of the treatment. When the former is smaller than the latter, treatment can be evaluated effective. When the former is equivalent to or larger than the latter, in contrast, treatment can be evaluated ineffective or insufficient. On the basis of the results of evaluation, continuation of treatment, modification of treatment policy, discontinuation of treatment, and the like can be determined.

The results of quantification of the antibody in the specimen obtained from the test subject subjected to treatment of ulcerative colitis or primary sclerosing cholangitis obtained in the step of detection in the method for evaluating therapeutic effects according to the present description are compared with, for example, the results of quantification of the antibody in the specimen obtained from a healthy individual of the same species as the test subject. When the former is equivalent to or smaller than the latter, treatment can be evaluated effective. When the former is larger than the latter, in contrast, treatment can be evaluated ineffective or insufficient. On the basis of the results of evaluation, continuation of treatment, modification of treatment policy, discontinuation of treatment, and the like can be determined.

### <Method for screening for candidate substance of therapeutic agent for ulcerative colitis or primary sclerosing cholangitis>

The first method for screening according to the present description relates to a method for screening for a candidate substance of a therapeutic agent for ulcerative colitis comprising:
a step of antibody detection comprising detecting an antibody immunologically reacting with full-length or a fragment of integrin αVβ6 and/or an antibody immunologically reacting with full-length or a fragment of integrin αVβ3 in a specimen obtained from a subject to which the test substance is administered; and
a step of selection comprising selecting the test substance as a candidate substance of a therapeutic agent for ulcerative colitis when the amount of the antibody in the specimen is decreased upon administration of the test substance.

The second method for screening according to the present description relates to a method for screening for a candidate substance of a therapeutic agent for primary sclerosing cholangitis comprising:
a step of antibody detection comprising detecting an antibody immunologically reacting with full-length or a fragment of integrin αVβ6 and/or an antibody immunologically reacting with full-length or a fragment of integrin αVβ3 in a specimen obtained from a subject to which the test substance is administered; and
a step of selection comprising selecting the test substance as a candidate substance of a therapeutic agent for primary sclerosing cholangitis when the amount of the antibody in the specimen is decreased upon administration of the test substance.

As described above, an antibody immunologically reacting with full-length or a fragment of integrin αVβ6 and an antibody immunologically reacting with full-length or a fragment of integrin αVβ3 in the specimen are useful as indicators of ulcerative colitis and primary sclerosing cholangitis. When the amount of the antibody in the specimen is decreased upon administration of the test substance, accordingly, the test substance can be selected as a candidate substance of a therapeutic agent for ulcerative colitis or primary sclerosing cholangitis.

In the embodiment comprising the step of antibody detection in the method for screening according to the present description, specific embodiments of the specimen, the full-length or a fragment of integrin αVβ6, the full-length or a fragment of integrin αVβ3, the antibody, the step of detection, and the like are as described above with regard to the test method according to the present invention.

In the method for screening according to the present description, a test substance is not particularly limited, provided that it can be a candidate of a novel medicine. Typically, a subject to which the test substance is administered is, for example, a non-human animal model of ulcerative colitis or primary sclerosing cholangitis exhibiting a larger amount of an antibody immunologically reacting with full-length or a fragment of integrin αVβ6 and/or full-length or a fragment of integrin αVβ3 in the specimen, compared with a healthy subject.

In the step of selection in the method for screening according to the present description, the results of quantification of the antibody in the specimen obtained from a subject to which the test substance is administered obtained in the step of antibody detection are compared with, for example, the results of quantification of the antibody in the specimen obtained from the same subject before administration of the test substance. When the former is smaller than the latter, the amount of the antibody in the specimen is determined to have decreased upon administration of the test substance, and the test substance can be selected as a candidate substance of a therapeutic agent for ulcerative colitis or primary sclerosing cholangitis.

In the step of selection in the method for screening according to the present description, the results of quantification of the antibody in the specimen obtained from a subject to which the test substance is administered obtained in the step of antibody detection are compared with, for example, the results of quantification of the antibody in the specimen obtained from a healthy individual of the same species as the subject. When the former is equivalent to or smaller than the latter, the amount of the antibody in the specimen is determined to have decreased upon administration of the test substance, and the test substance can be selected as a candidate substance of a therapeutic agent for ulcerative colitis or primary sclerosing cholangitis.

### <Method for preparing non-human animal model of ulcerative colitis or primary sclerosing cholangitis>

The first method for preparing a non-human animal model according to the present description relates to a method for preparing a non-human animal model of ulcerative colitis comprising at least one of:
a step of antibody administration comprising administering an antibody immunologically reacting with full-length or a fragment of integrin αVβ6 and/or an antibody immunologically reacting with full-length or a fragment of integrin αVβ3 to a non-human animal; and
a step of immunization comprising immunizing a non-human animal with full-length or a fragment of integrin αVβ6 and/or full-length or a fragment of integrin αVβ3 as an antigen.

The second method for preparing a non-human animal model according to the present description relates to a method for preparing a non-human animal model of primary sclerosing cholangitis comprising at least one of:
a step of antibody administration comprising administering an antibody immunologically reacting with full-length or a fragment of integrin αVβ6 and/or an antibody immunologically reacting with full-length or a fragment of integrin αVβ3 to a non-human animal; and
a step of immunization comprising immunizing a non-human animal with full-length or a fragment of integrin αVβ6 and/or full-length or a fragment of integrin αVβ3 as an antigen.

Concerning the method for preparing a non-human animal model according to the present description, specific embodiments of the full-length or a fragment of integrin αVβ6, the full-length or a fragment of integrin αVβ3, the antibody, and the like are as described above with regard to the test method according to the present invention.

As a result of the step of antibody administration comprising administering an antibody immunologically reacting with full-length or a fragment of integrin αVβ6 and/or full-length or a fragment of integrin αVβ3 to a non-human animal, a non-human animal model of ulcerative colitis or primary sclerosing cholangitis can be prepared.

Examples of non-human animals to which the antibody is administered include mice, such as BALB/c mice and B6 mice, and other non-human animals.

In the step of antibody administration, the route of administration of an antibody immunologically reacting with full-length or a fragment of integrin αVβ6 and/or an antibody immunologically reacting with full-length or a fragment of integrin αVβ3 is not particularly limited. For example, administration can be performed via a hypodermic, intraperitoneal, or intravenous route. The antibody is not necessarily administered to a non-human animal in the form of a purified antibody. For example, an antibody-containing serum may be administered to a non-human animal.

The antibody to be administered in the step of antibody administration is selected in a manner such that the antibody can immunologically react with integrin αVβ6 and/or integrin αVβ3 that is present in a non-human animal to which the antibody is to be administered.

As a result of the step of immunization comprising immunizing a non-human animal with full-length or a fragment of integrin αVβ6 and/or full-length or a fragment of integrin αVβ3 as an antigen, a non-human animal model of ulcerative colitis or primary sclerosing cholangitis can be prepared.

Examples of non-human animals to which the antigen is administered include mice, such as BALB/c mice and B6 mice, and other non-human animals.

A method of immunization is not particularly limited. Full-length or a fragment of integrin αVβ6 and/or full-length or a fragment of integrin αVβ3 is administered to a non-human animal together with an adequate adjuvant such as complete Freund's adjuvant via a hypodermic, intravenous, or intraperitoneal route. Thus, the non-human animal can be immunized.

Full-length or a fragment of integrin αVβ6 and/or full-length or a fragment of integrin αVβ3 to be administered as an antigen in the step of immunization is selected in a manner such that the antibody to such antigen can, because of autoimmunity, immunologically react with the integrin αVβ6 and/or integrin αVβ3 that is present in the non-human animal to be immunized. Examples

### <Experiment 1: Identification of autoantibody specific to patient with ulcerative colitis>

The present inventors had intended to identify the antigen of the autoantibody specific to a patient with ulcerative colitis. To this end, they had selected known extracellular matrix proteins as candidate antigens from the literature, immobilized each of such proteins on a solid phase, and examined as to the presence or absence of an antibody binding to the extracellular matrix protein in a serum sample obtained from a patient with ulcerative colitis via enzyme-linked immunosorbent assay (ELISA).

In ELISA, the target autoantibody was human IgG that binds to a candidate antigen immobilized on a solid phase that can be present in the serum of a patient with ulcerative colitis, and the detection antibody was the rabbit anti-human IgG polyclonal antibody labeled with horseradish peroxidase (HRP).

### 1. Candidate antigens

As candidate antigens, the proteins described below, known as extracellular matrix proteins, were used.
Human integrin α2bβ3 (7148-A2, R&D Systems)
Human integrin α1β1 (7064-AB, R&D Systems)
Human integrin α2β1 (5698-A2, R&D Systems)
Human integrin α3β1 (2840-A3, R&D Systems)
Human integrin α4β1 (5668-A4, R&D Systems)
Human integrin α4β7 (5397-A3, R&D Systems)
Human integrin α5β1 (3230-A5, R&D Systems)
Human integrin α6β1 (7809-A6, R&D Systems)
Human integrin α6β4 (5497-A6, R&D Systems)
Human integrin α9β1 (5438-A9, R&D Systems)
Human integrin α10β1 (5895-AB, R&D Systems)
Human integrin α11β1 (6357-AB, R&D Systems)
Human integrin αEβ7 (5850-A3, R&D Systems)
Human integrin αLβ2 (3868-AV, R&D Systems)
Human integrin αMβ2 (4047-AM, R&D Systems)
Human integrin αVβ1 (6579-AVB, R&D Systems)
Human integrin αVβ3 (3050-AV, R&D Systems)
Human integrin αVβ5 (2528-AV, R&D Systems)
Human integrin αVβ6 (3817-AV, R&D Systems)
Human integrin αVβ8 (4135-AV, R&D Systems)
Human integrin αXβ2 (5755-AX, R&D Systems)

### 2. Serum samples

Serum samples were obtained from 8 patients who were diagnosed to have ulcerative colitis.

Patients having ulcerative colitis not complicated with primary sclerosing cholangitis were selected.

As the control serum samples, serum samples were obtained from 3 healthy subjects.

### 3. Procedures

In the following test, the ELISA Starter Accessory Kit (E101, Bethyl Laboratories) was used, unless otherwise specified.

The ELISA coating buffer, the ELISA wash solution, the ELISA blocking buffer, and the conjugate diluent were prepared in accordance with the instructions of the kit.

All steps were performed at room temperature, unless otherwise specified.

### 3.1. Coating with candidate antigen

(1) One of the candidate antigens described above was diluted with the ELISA coating buffer of the kit to prepare a 2 µg/ml solution thereof. The solution was applied at 100 µl/well to the microwell plate of the kit.
(2) The microwell plate was subjected to incubation at 4°C for 60 minutes.
(3) After the incubation, the solution was suction-removed from each well.
(4) Each well was washed with the ELISA wash solution of the kit. Specifically, each well was filled with the ELISA wash solution, and the ELISA wash solution was suction-removed (i.e., washing). This procedure of washing was repeated 3 times.

### 3.2. Blocking

(1) The ELISA blocking buffer of the kit was added at 200 µl/well.
(2) Incubation was carried out for 30 minutes.
(3) After the incubation, the ELISA blocking buffer was removed, and each well was washed 3 times.

### 3.3. Test sample

(1) The serum samples of patients with ulcerative colitis (n = 8) or the control serum samples (n = 3) were diluted with the conjugate diluent of the kit at 1:100.
(2) The diluted serum samples obtained were added at 100 µl/well to the microwell plate after the blocking.
(3) Incubation was carried out for 60 minutes.
(4) After the incubation, the diluted serum samples were removed, and each well was washed 5 times.

### 3.4. HRP-conjugated detection antibody

(1) The horseradish peroxidase (HRP)-conjugated detection antibody (abcam6759, rabbit anti-human IgG H&L (HRP)) was diluted with the conjugate diluent of the kit at 1:50,000.
(2) The diluted detection antibody solution obtained above was added at 100 µl/well to the microwell plate with which the serum had been brought into contact.
(3) Incubation was carried out for 60 minutes.
(4) After the incubation, the diluted detection antibody solution was removed, and each well was washed 5 times.

### 3.5. Enzyme substrate reaction

(1) Under the conditions recommended by the manufacturer, a TMB (3,3',5,5'-tetramethylbenzidine) solution was prepared.
(2) The TMB solution was added at 100 µl/well to the microwell plate with which the detection antibody had been brought into contact.
(3) Incubation was carried out for 7 to 8 minutes.
(4) In order to terminate TMB oxidation, 0.18 M H₂SO₄ was added at 100 µl/well.
(5) With the use of a microplate reader, color development caused by the product of the oxidation reaction was assayed at 450 nm.

### 4. Results and discussion

The results are shown in Figs. 1-1 to 1-4. The title of each chart shows a combination of an integrin α subunit type and a β subunit type of a candidate antigen.

In Figs. 1-1 to 1-4, the vertical axis indicates the relative absorbance at 450 nm proportional to the amount of the detection antibody on a solid phase. "UC" indicates the serum samples of patients with ulcerative colitis (n = 8), and "Control" indicates the control serum samples (n = 3).

When human integrin αVβ6 and human integrin αVβ3 were used as antigens, the concentration of the antibodies to the antigens were found to be higher in the 4 serum samples of patients with ulcerative colitis than in the control serum samples (Fig. 1-3, Fig. 1-4). This indicates that an increase in the concentration of the autoantibody to human integrin αVβ6 in the serum and an increase in the concentration of the autoantibody to human integrin αVβ3 in the serum can serve as indicators for diagnosis of ulcerative colitis. When other human integrins were used as antigens, there was no significant difference in the concentration of the antibodies to the antigens between the serum samples of patients with ulcerative colitis and the control serum samples.

### <Experiment 2: Diagnosis of ulcerative colitis based on the anti-human integrin αVβ6 antibody>

It was confirmed in Experiment 1 that the anti-human integrin αVβ6 antibody concentration was high in the serum samples of patients with ulcerative colitis.
In order to examine whether or not ulcerative colitis would be diagnosed based on the anti-human integrin αVβ6 antibody concentration in the serum, the number of samples was increased in this experiment. Specifically, serum samples obtained from patients with ulcerative colitis (n = 63), serum samples obtained from patients diagnosed to have Crohn's disease (n = 48), and control serum samples obtained from healthy subjects or patients diagnosed to have Behcet's disease, eosinophilic gastroenteritis, infectious enteritis, ischemic enteritis, Cronkhite-Canada syndrome, or inflammatory bowel disease unclassified (IBDU) (n = 11) were used.

In this experiment, also, patients with ulcerative colitis not complicated with primary sclerosing cholangitis were selected.

The anti-human integrin αVβ6 antibody concentration in the serum samples was assayed via ELISA in the same manner as in Experiment 1, except that CaCl₂ and MgCl₂ were added to the final concentration of 1 mM to the ELISA wash solution, the ELISA blocking buffer, and the conjugate diluent.

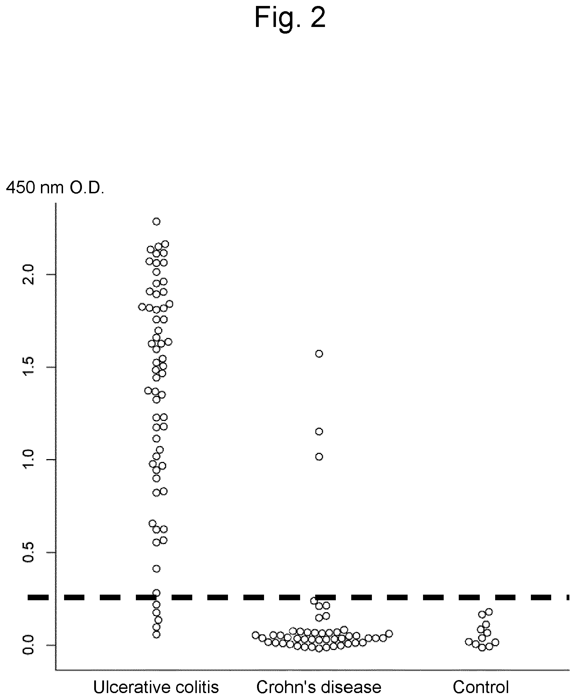

The results are shown in Fig. 2. In Fig. 2, the vertical axis indicates the relative absorbance at 450 nm proportional to the amount of the detection antibody on a solid phase. "Ulcerative colitis" indicates the serum samples of patients with ulcerative colitis (n = 63), "Crohn's disease" indicates the serum samples of patients with Crohn's disease (n = 48), and "Control" indicates the control serum samples (n = 11). A cut-off value indicated by a broken line is a sum of the average relative absorbance of the control serum samples and a value that is 3 times the standard deviation (SD).

As shown in Fig. 2, 58 samples out of the 63 serum samples of patients with ulcerative colitis were found positive for the anti-human integrin αVβ6 antibody (i.e., sensitivity: 92%). In contrast, 45 samples out of the 48 serum samples of patients with Crohn's disease were negative therefor, and 11 out of the 11 control serum samples were negative therefor (i.e., specificity: 95%). The results demonstrate that ulcerative colitis can be diagnosed based on the concentration of the autoantibody to human integrin αVβ6 in the blood.

### <Experiment 3: Diagnosis of primary sclerosing cholangitis based on the anti-human integrin αVβ6 antibody>

This experiment examined whether or not primary sclerosing cholangitis (PSC) could be diagnosed based on the anti-human integrin αVβ6 antibody concentration in the serum. Samples used in this experiment included: serum samples obtained from patients with PSC (n = 24), serum samples obtained from patients diagnosed to have IgG4-related sclerosing cholangitis (n = 5), and control serum samples obtained from healthy subjects (n = 6).

Among the 24 patients of primary sclerosing cholangitis, 18 patients had PSC complicated with ulcerative colitis.

The anti-human integrin αVβ6 antibody concentration in the serum samples was assayed via ELISA in the same manner as in Experiment 1, except that CaCl₂ and MgCl₂ were added to the final concentration of 1 mM to the ELISA wash solution, the ELISA blocking buffer, and the conjugate diluent.

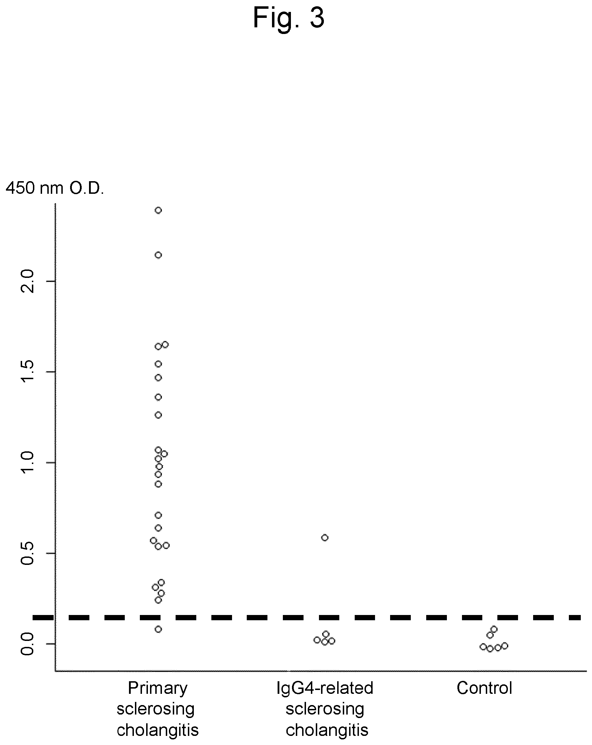

The results are shown in Fig. 3. In Fig. 3, the vertical axis indicates the relative absorbance at 450 nm proportional to the amount of the detection antibody on a solid phase. "Primary sclerosing cholangitis" indicates the serum samples of patients with PSC (n = 24), "IgG4-related sclerosing cholangitis" indicates the serum samples of patients with IgG4-related sclerosing cholangitis (n = 5), and "Control" indicates the control serum samples (n = 6). A cut-off value indicated by a broken line is a sum of the average relative absorbance of the control serum samples and a value that is 3 times the standard deviation (SD).

As shown in Fig. 3, 23 samples out of the 24 serum samples of patients with PSC were found positive for the anti-human integrin αVβ6 antibody (i.e., sensitivity: 96%), 4 samples out of the 5 serum samples of patients with IgG4-related sclerosing cholangitis were negative therefor, and 6 samples out of the 6 control serum samples were negative therefor (i.e., specificity: 91%). The results demonstrate that primary sclerosing cholangitis (PSC) can be diagnosed based on the concentration of the autoantibody to human integrin αVβ6 in the blood.

### <Experiment 4: Diagnosis of ulcerative colitis based on the anti-human integrin αVβ3 antibody>

It was confirmed in Experiment 1 that the anti-human integrin αVβ3 antibody concentration was high in the serum samples of patients with ulcerative colitis.

In order to examine whether or not ulcerative colitis would be diagnosed based on the anti-human integrin αVβ3 antibody concentration in the serum the number of samples was increased in this experiment. Specifically, serum samples obtained from patients with ulcerative colitis (n = 15), serum samples obtained from patients diagnosed to have Crohn's disease (n = 4), and control serum samples obtained from healthy subjects (n = 9) were used.

In this experiment, also, patients with ulcerative colitis not complicated with primary sclerosing cholangitis were selected.

The anti-human integrin αVβ3 antibody concentration in the serum samples was assayed via ELISA in the same manner as in Experiment 1, except that CaCl₂ and MgCl₂ were added to the final concentration of 1 mM to the ELISA wash solution, the ELISA blocking buffer, and the conjugate diluent.

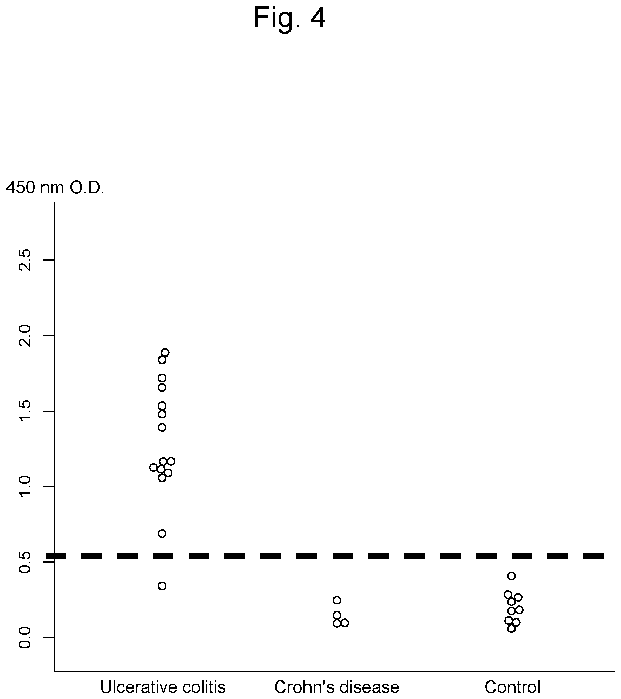

The results are shown in Fig. 4. In Fig. 4, the vertical axis indicates the relative absorbance at 450 nm proportional to the amount of the detection antibody on a solid phase. "Ulcerative colitis" indicates the serum samples of patients with ulcerative colitis (n = 15), "Crohn's disease" indicates the serum samples of patients with Crohn's disease (n = 4), and "Control" indicates the control serum samples (n = 9). A cut-off value indicated by a broken line is a sum of the average relative absorbance of the control serum samples and a value that is 3 times the standard deviation (SD).

As shown in Fig. 4, 58 samples out of the 63 serum samples of patients with ulcerative colitis were found positive for the anti-human integrin αVβ3 antibody (i.e., sensitivity: 93%). In contrast, 4 samples out of the 4 serum samples of patients with Crohn's disease were negative therefor, and 9 out of the 9 control serum samples were negative therefor (i.e., specificity: 100%). The results demonstrate that ulcerative colitis can be diagnosed based on the concentration of the autoantibody to human integrin αVβ3 in the blood.

### <Experiment 5: Diagnosis of primary sclerosing cholangitis based on the anti-human integrin αVβ3 antibody>

This experiment examined whether or not primary sclerosing cholangitis (PSC) would be diagnosed based on the anti-human integrin αVβ3 antibody concentration in the serum. Samples used in this experiment included: serum samples obtained from patients with PSC (n = 24), serum samples obtained from patients diagnosed to have IgG4-related sclerosing cholangitis (n = 5), and control serum samples obtained from healthy subjects or patients diagnosed to have bile duct cancer, primary biliary cholangitis, cirrhosis, or liver cell carcinoma (n = 6).

Among the 24 patients of primary sclerosing cholangitis, 18 patients had PSC complicated with ulcerative colitis.

The anti-human integrin αVβ3 antibody concentration in the serum samples was assayed via ELISA in the same manner as in Experiment 1, except that CaCl₂ and MgCl₂ were added to the final concentration of 1 mM to the ELISA wash solution, the ELISA blocking buffer, and the conjugate diluent.

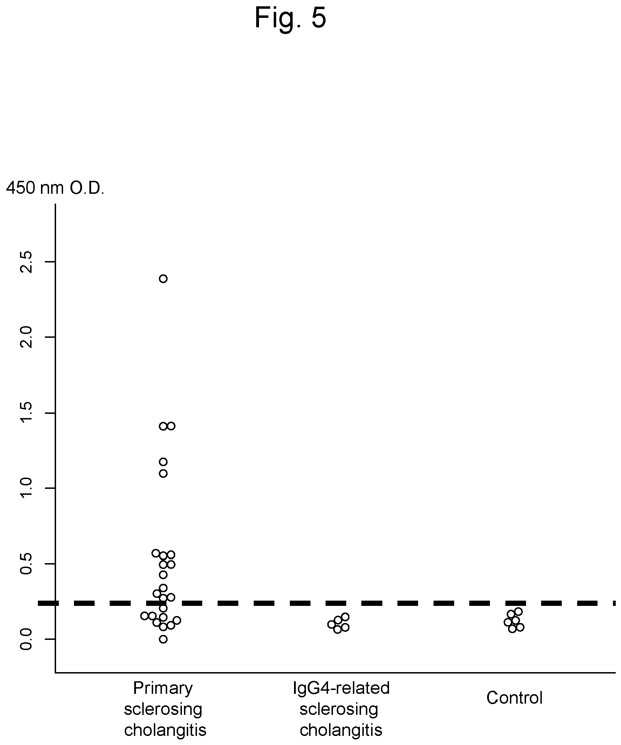

The results are shown in Fig. 5. In Fig. 5, the vertical axis indicates the relative absorbance at 450 nm proportional to the amount of the detection antibody on a solid phase. "Primary sclerosing cholangitis" indicates the serum samples of patients with PSC (n = 24), "IgG4-related sclerosing cholangitis" indicates the serum samples of patients with IgG4-related sclerosing cholangitis (n = 5), and "Control" indicates the control serum samples (n = 6). A cut-off value indicated by a broken line is a sum of the average relative absorbance of the control serum samples and a value that is 3 times the standard deviation (SD).

As shown in Fig. 5, 15 samples out of the 24 serum samples of patients with PSC were found positive for the anti-human integrin αVβ3 antibody (i.e., sensitivity: 63%). In contrast, 5 samples out of the 5 serum samples of patients with IgG4-related sclerosing cholangitis were negative therefor, and 6 samples out of the 6 control serum samples were negative therefor (i.e., specificity: 100%). The results demonstrate that primary sclerosing cholangitis (PSC) can be diagnosed based on the concentration of the autoantibody to human integrin αVβ3 in the blood.

### <Experiment 6: Diagnosis of ulcerative colitis based on the anti-human integrin αVβ6 antibody>

This experiment confirmed that the method for diagnosis of ulcerative colitis based on the anti-human integrin αVβ6 antibody concentration in the serum would enable specific diagnosis of ulcerative colitis. Samples used in this experiment included: serum samples of patients with ulcerative colitis (n = 66), serum samples of patients with Crohn's disease (n = 47), and serum samples of patients with other intestinal diseases (infectious enteritis, ischemic enteritis, Behcet's disease, eosinophilic gastroenteritis, Cronkhite-Canada syndrome, or IBDU) (n = 9).

In this experiment, also, patients with ulcerative colitis not complicated with primary sclerosing cholangitis were selected.

The anti-human integrin αVβ6 antibody concentration in the serum samples was assayed via ELISA in the same manner as in Experiment 2.

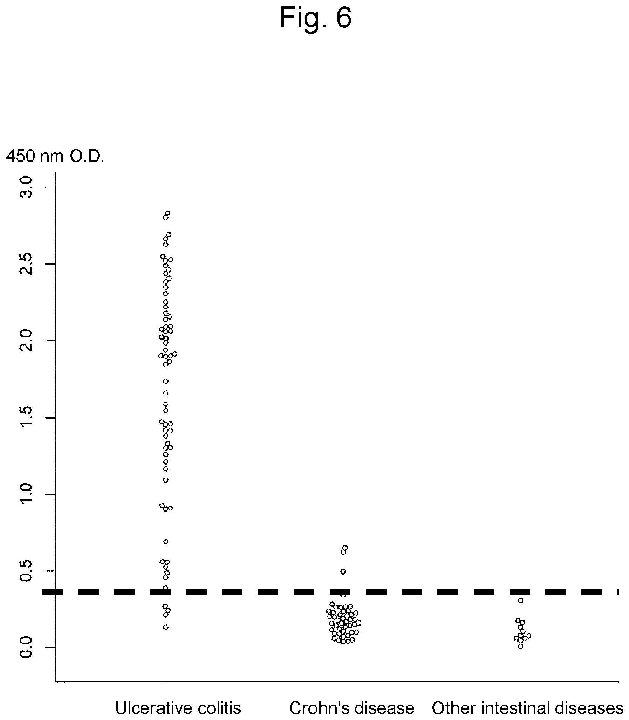

The results are shown in Fig. 6. In Fig. 6, the vertical axis indicates the relative absorbance at 450 nm proportional to the amount of the detection antibody on a solid phase. "Ulcerative colitis" indicates the serum samples of patients with ulcerative colitis, "Crohn's disease" indicates the serum samples of patients with Crohn's disease, and "Other intestinal diseases" indicates the serum samples of patients with other intestinal diseases. A cut-off value indicated by a broken line is a sum of the average relative absorbance of the serum samples of patients with other intestinal diseases and a value that is 3 times the standard deviation (SD).

Concerning the anti-human integrin αVβ6 antibody, 62 samples out of the 66 serum samples of patients with ulcerative colitis were found positive therefor (i.e., sensitivity: 94%). In contrast, 44 samples out of the 47 serum samples of patients with Crohn's disease were negative therefor, and 9 out of the 9 serum samples of patients with other intestinal diseases were negative therefor (i.e., specificity: 95%). The results demonstrate that the concentration of the autoantibody to human integrin αVβ6 in the blood is specifically high in patients with ulcerative colitis.

### <Experiment 7: Diagnosis of ulcerative colitis based on the anti-human integrin αVβ6 antibody>

This experiment confirmed that the method for diagnosis of ulcerative colitis based on the anti-human integrin αVβ6 antibody concentration in the serum would enable specific diagnosis of ulcerative colitis. Samples used in this experiment included: serum samples of patients with ulcerative colitis (n = 51), serum samples of patients with Crohn's disease (n = 26), serum samples of patients with other intestinal diseases (infectious enteritis, ischemic enteritis, Behcet's disease, eosinophilic gastroenteritis, Cronkhite-Canada syndrome, or IBDU) (n = 24), the serum samples of patients with collagen disease (n = 27), and serum samples of healthy subjects (n = 22).

In this experiment, also, patients with ulcerative colitis not complicated with primary sclerosing cholangitis were selected.

The anti-human integrin αVβ6 antibody concentration in the serum samples was assayed via ELISA in the same manner as in Experiment 2.

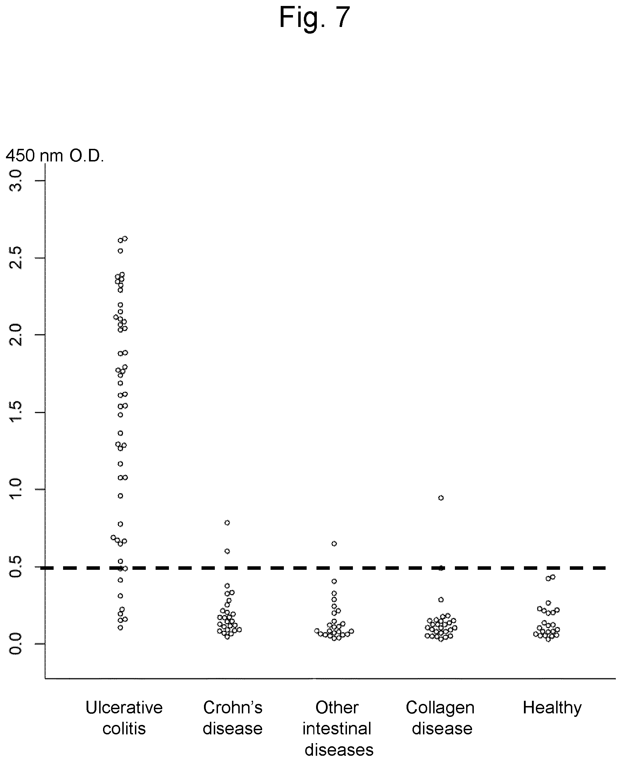

The results are shown in Fig. 7. In Fig. 7, the vertical axis indicates the relative absorbance at 450 nm proportional to the amount of the detection antibody on a solid phase. "Ulcerative colitis" indicates the serum samples of patients with ulcerative colitis, "Crohn's disease" indicates the serum samples of patients with Crohn's disease, "Other intestinal diseases" indicates the serum samples of patients with other intestinal diseases, "Collagen disease" indicates the serum samples of patients with collagen disease, and "Healthy" indicates the serum samples of healthy subjects. A cut-off value indicated by a broken line is a sum of the average relative absorbance of the serum samples of healthy subjects and a value that is 3 times the standard deviation (SD).

Concerning the anti-human integrin αVβ6 antibody, 43 samples out of the 51 serum samples of patients with ulcerative colitis were found positive therefor (i.e., sensitivity: 84%). In contrast, 24 samples out of the 26 serum samples of patients with Crohn's disease were found negative therefor, 23 samples out of the 24 serum samples of patients with other intestinal diseases were found negative therefor, 26 samples out of the 27 serum samples of patients with collagen disease were found negative therefor, and 22 samples out of the 22 serum samples of healthy subjects were found negative therefor (i.e., specificity: 96%). The results demonstrate that the concentration of the autoantibody to human integrin αVβ6 in the blood is specifically high in patients with ulcerative colitis as in the case of Experiment 6.

### <Experiment 8: IgG subclass of anti-human integrin αVβ6 autoantibody in patient with ulcerative colitis>

This experiment studied the IgG subclasses of the anti-human integrin αVβ6 autoantibody in the serum samples obtained from patients with ulcerative colitis.

Samples used in this experiment included: serum samples of patients with ulcerative colitis (n = 47), serum samples of patients with Crohn's disease (n = 8), and serum samples of patients with other intestinal diseases (infectious enteritis, ischemic enteritis, Behcet's disease, eosinophilic gastroenteritis, Cronkhite-Canada syndrome, or IBDU) (n = 8).

In this experiment, also, patients with ulcerative colitis not complicated with primary sclerosing cholangitis were selected.

In order to detect IgG1, IgG2, IgG3, and IgG4 that specifically bind to human integrin αVβ6 while distinguishing them from one another, the ELISA technique involving the use of human integrin αVβ6 as an antigen described in Experiment 1 was performed in the same manner herein, except that the HRP-conjugated anti-human IgG1 antibody (Anti-IgG1-HRP, AP006, Binding Site), the HRP-conjugated anti-human IgG2 antibody (Anti-IgG2-HRP, AP007, Binding Site), the HRP-conjugated anti-human IgG3 antibody (Anti-IgG3-HRP, AP008, Binding Site), or the HRP-conjugated anti-human IgG4 antibody (Anti-IgG4-HRP, AP009, Binding Site) was used as the detection antibody and CaCl₂ and MgCl₂ were added to the final concentration of 1 mM to the ELISA wash solution, the ELISA blocking buffer, and the conjugate diluent.

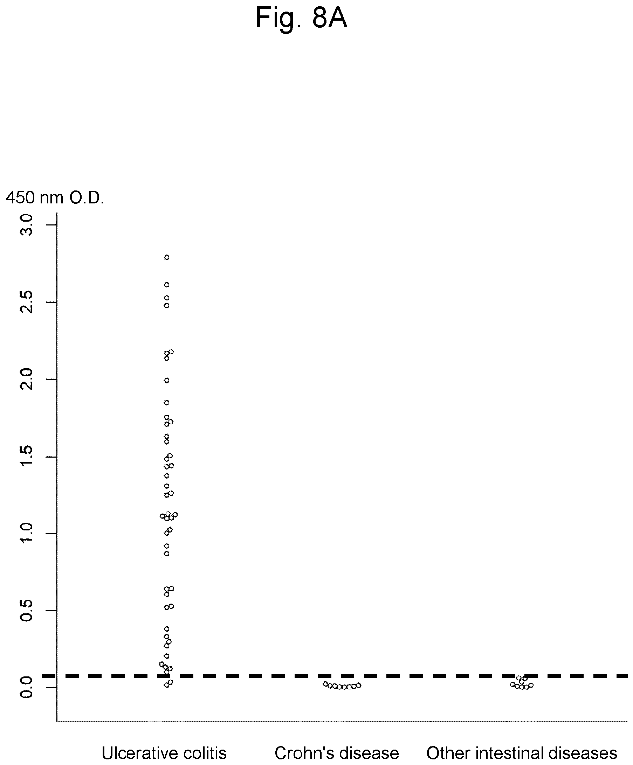

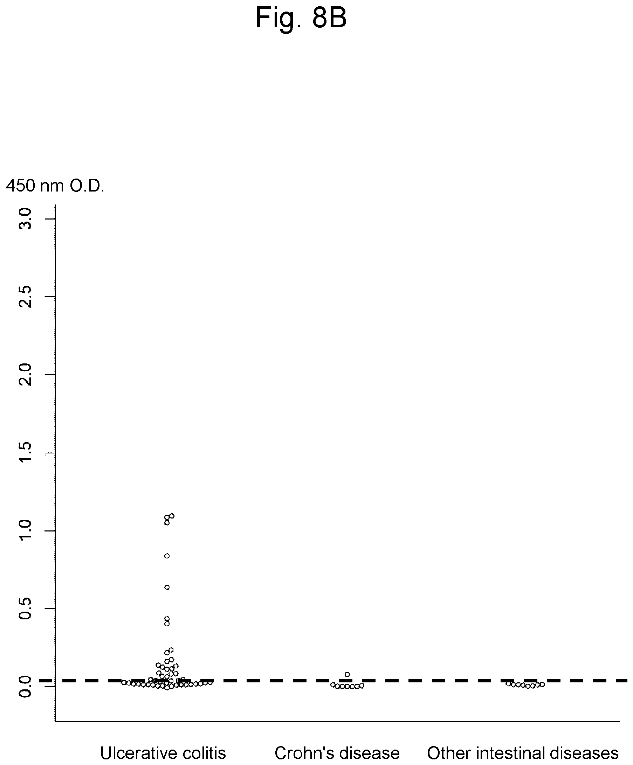

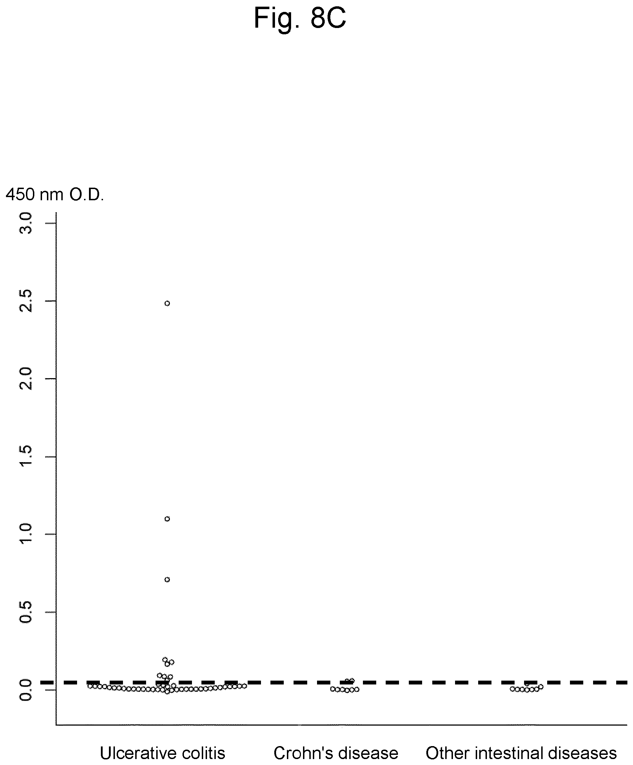

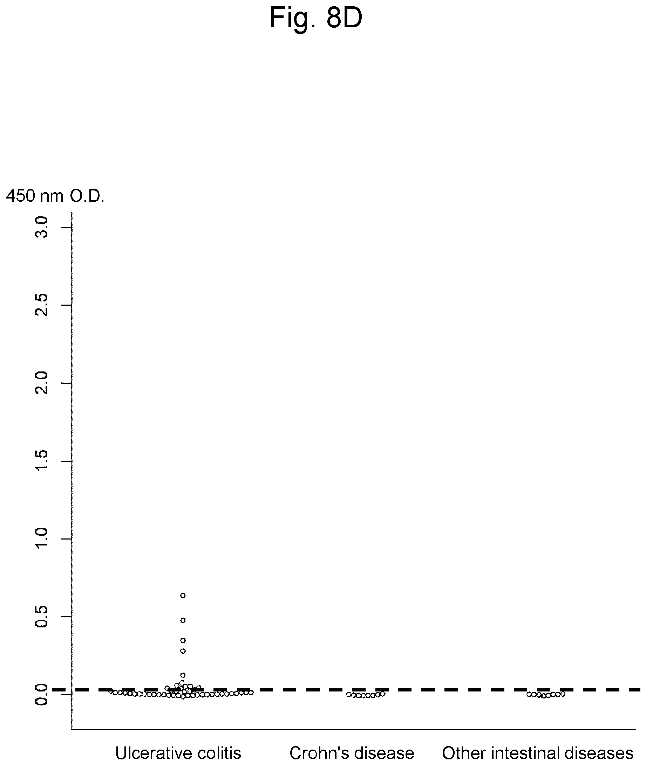

Fig. 8A shows the results of IgG1 detection, Fig. 8B shows the results of IgG2 detection, Fig. 8C shows the results of IgG3 detection, and Fig. 8D shows the results of IgG4 detection. In Fig. 8A to Fig. 8D, the vertical axis indicates the relative absorbance at 450 nm proportional to the amount of the detection antibody on a solid phase. "Ulcerative colitis" indicates the serum samples of patients with ulcerative colitis (n = 47), "Crohn's disease" indicates the serum samples of patients with Crohn's disease (n = 8), and "Other intestinal diseases" indicates the serum samples of patients with other intestinal diseases (n = 8). In Fig. 8A to Fig. 8D, a cut-off value indicated by a broken line is a sum of the average relative absorbance of the serum samples of patients with other intestinal diseases and a value that is 3 times the standard deviation (SD).

Fig. 8A shows the results described below. That is, 45 samples out of the 47 serum samples of patients with ulcerative colitis were positive for IgG1 that binds to human integrin αVβ6 (i.e., sensitivity: 96%), 8 samples out of the 8 serum samples of patients with Crohn's disease were negative therefor, and 8 samples out of the 8 serum samples of patients with other intestinal diseases were negative therefor.

Fig. 8B shows the results described below. That is, 29 samples out of the 47 serum samples of patients with ulcerative colitis were positive for IgG2 that binds to human integrin αVβ6 (i.e., sensitivity: 62%), 7 samples out of the 8 serum samples of patients with Crohn's disease were negative therefor, and 8 samples out of the 8 serum samples of patients with other intestinal diseases were negative therefor.

Fig. 8C shows the results described below. That is, 10 samples out of the 47 serum samples of patients with ulcerative colitis were positive for IgG3 that binds to human integrin αVβ6 (i.e., sensitivity: 21%), 6 samples out of the 8 serum samples of patients with Crohn's disease were negative therefor, and 8 samples out of the 8 serum samples of patients with other intestinal diseases were negative therefor.

Fig. 8D shows the results described below. That is, 19 samples out of the 47 serum samples of patients with ulcerative colitis were positive for IgG4 that binds to human integrin αVβ6 (i.e., sensitivity: 40%), 8 samples out of the 8 serum samples of patients with Crohn's disease were negative therefor, and 8 samples out of the 8 serum samples of patients with other intestinal diseases were negative therefor.

The results of experiment demonstrate that IgG1 is the dominant IgG subclass of the anti-human integrin αVβ6 autoantibody in a patient with ulcerative colitis.

### <Experiment 9: Isotype of the anti-human integrin αVβ6 autoantibody in patient with ulcerative colitis>

In this experiment, isotypes other than IgG; i.e., IgA, IgM, and IgE, were detected as the anti-human integrin αVβ6 autoantibodies in the serum samples of patients with ulcerative colitis.

Samples used in this experiment included: serum samples of patients with ulcerative colitis (n = 47), serum samples of patients with Crohn's disease (n = 8), and serum samples of patients with other intestinal diseases (infectious enteritis, ischemic enteritis, Behcet's disease, eosinophilic gastroenteritis, Cronkhite-Canada syndrome, or IBDU) (n = 8).

In this experiment, also, patients with ulcerative colitis not complicated with primary sclerosing cholangitis were selected.

In order to detect IgA, IgM, and IgE that specifically bind to human integrin αVβ6, the ELISA technique involving the use of human integrin αVβ6 as an antigen described in Experiment 1 was performed in the same manner herein, except that the HRP-conjugated anti-human IgA antibody (Goat anti-Human IgA Antibody HRP Conjugated, A80-102P, BETHYL), the HRP-conjugated anti-human IgM antibody (Goat anti-Human IgM Antibody HRP Conjugated, A80-100P, BETHYL), or the HRP-conjugated anti-human IgE antibody (Goat anti-Human IgE Cross-Adsorbed Secondary Antibody, HRP, A18799, Invitrogen) was used as the detection antibody and CaCl₂ and MgCl₂ were added to the final concentration of 1 mM to the ELISA wash solution, the ELISA blocking buffer, and the conjugate diluent.

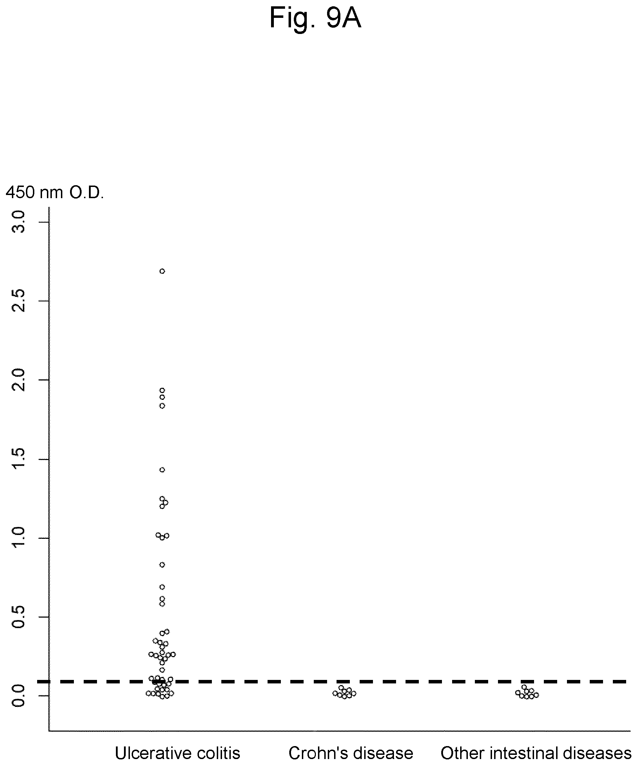

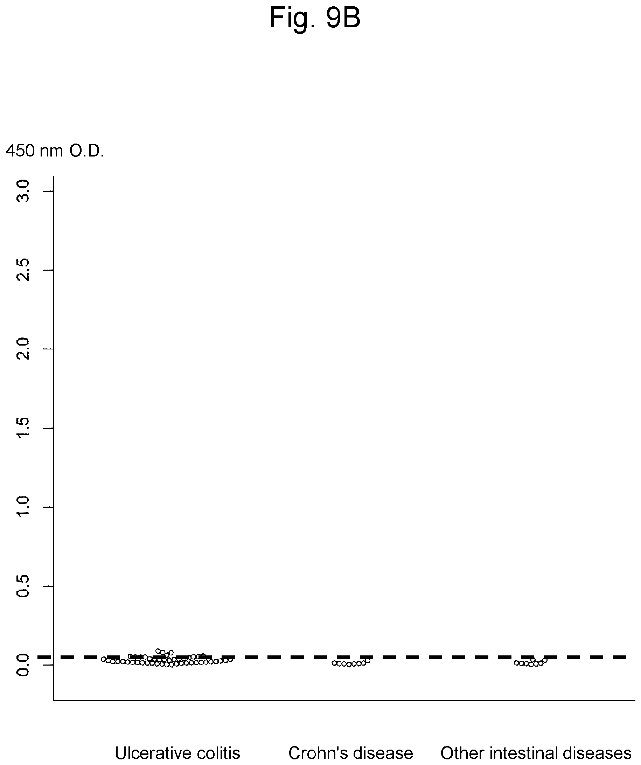

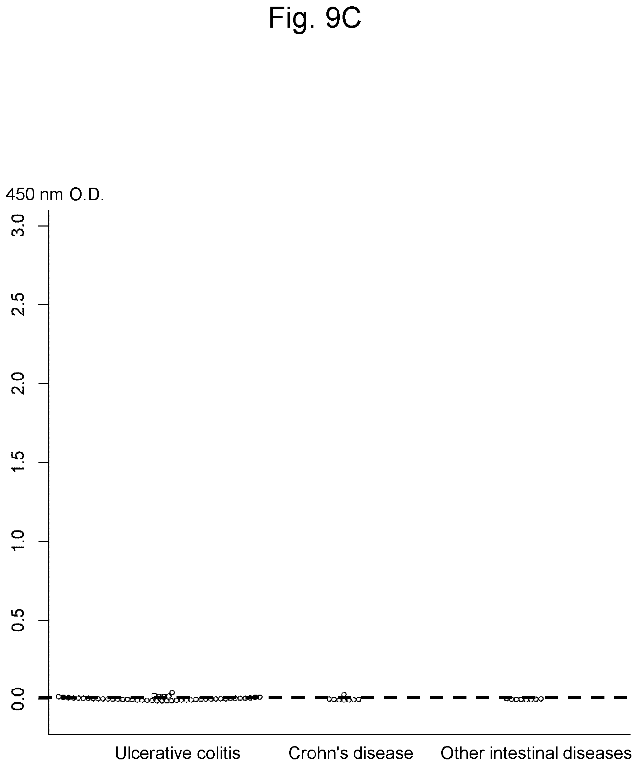

Fig. 9A shows the results of IgA detection, Fig. 9B shows the results of IgM detection, and Fig. 9C shows the results of IgE detection. In Fig. 9A to Fig. 9C, the vertical axis indicates the relative absorbance at 450 nm proportional to the amount of the detection antibody on a solid phase. "Ulcerative colitis" indicates the serum samples of patients with ulcerative colitis (n = 47), "Crohn's disease" indicates the serum samples of patients with Crohn's disease (n = 8), and "Other intestinal diseases" indicates the serum samples of patients with other intestinal diseases (n = 8). In Fig. 9A to Fig. 9C, a cut-off value indicated by a broken line is a sum of the average relative absorbance of the serum samples of patients with other intestinal diseases and a value that is 3 times the standard deviation (SD).

Fig. 9A shows the results described below. That is, 35 samples out of the 47 serum samples of patients with ulcerative colitis were positive for IgA that binds to human integrin αVβ6 (i.e., sensitivity: 74%), 8 samples out of the 8 serum samples of patients with Crohn's disease were negative therefor, and 8 samples out of the 8 serum samples of patients with other intestinal diseases were negative therefor.

Fig. 9B shows the results described below. That is, 11 samples out of the 47 serum samples of patients with ulcerative colitis were positive for IgM that binds to human integrin αVβ6 (i.e., sensitivity: 23%), 8 samples out of the 8 serum samples of patients with Crohn's disease were negative therefor, and 8 samples out of the 8 serum samples of patients with other intestinal diseases were negative therefor.

Fig. 9C shows the results described below. That is, 10 samples out of the 47 serum samples of patients with ulcerative colitis were positive for IgE that binds to human integrin αVβ6 (i.e., sensitivity: 21%), 7 samples out of the 8 serum samples of patients with Crohn's disease were negative therefor, and 8 samples out of the 8 serum samples of patients with other intestinal diseases were negative therefor.

The results of experiment demonstrate that, in addition to IgG, IgA, IgM, and IgE are present as the anti-human integrin αVβ6 autoantibodies in patients with ulcerative colitis and IgA is dominant.

### <Experiment 10: Correlation between ulcerative colitis progression and the antibody titer of the anti-human integrin αVβ6 autoantibody>

The correlation between ulcerative colitis progression and the antibody titer of the anti-human integrin αVβ6 autoantibody (IgG) was examined.

Disease progression was evaluated using the partial Mayo scores obtained by subtracting the mucosal observation (endoscopic observation) from the Mayo scores.

The antibody titer of the anti-human integrin αVβ6 autoantibody (IgG) was assayed in the same manner as in Experiment 2 above.

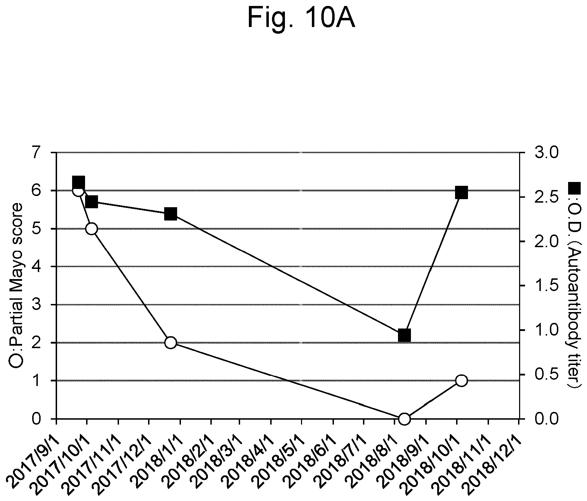

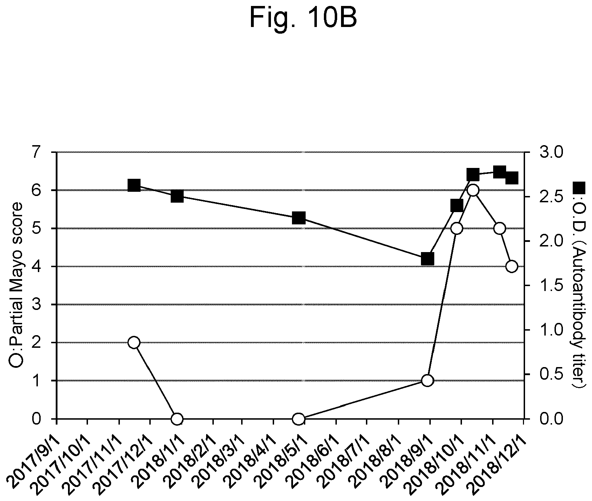

Fig. 10A and Fig. 10B show the partial Mayo scores of 2 representative patients with ulcerative colitis at each point during a period from November, 2017 to November, 2018 (the left vertical axis) and the antibody titers of the anti-human integrin αVβ6 autoantibody (IgG) (the right vertical axis).

In accordance with changes in the partial Mayo scores, the antibody titers were also changed in both patients.

### <Experiment 11: Diagnosis of ulcerative colitis based on the anti-human integrin αVβ3 antibody>

This experiment confirmed that the method for diagnosis of ulcerative colitis based on the anti-human integrin αVβ3 antibody concentration in the serum would enable specific diagnosis of ulcerative colitis. Samples used in this experiment included: serum samples of patients with ulcerative colitis (n = 66), serum samples of patients with Crohn's disease (n = 47), and serum samples of patients with other intestinal diseases (infectious enteritis, ischemic enteritis, Behcet's disease, eosinophilic gastroenteritis, Cronkhite-Canada syndrome, or IBDU) (n = 9).

In this experiment, also, patients with ulcerative colitis not complicated with primary sclerosing cholangitis were selected.

The anti-human integrin αVβ3 antibody concentration in the serum samples was assayed via ELISA in the same manner as in Experiment 4.

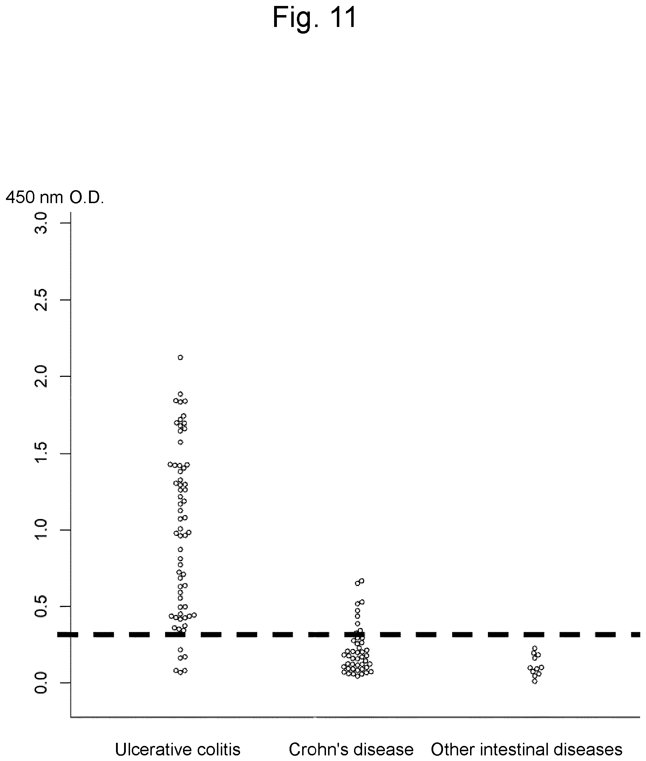

The results are shown in Fig. 11. In Fig. 11, the vertical axis indicates the relative absorbance at 450 nm proportional to the amount of the detection antibody on a solid phase. "Ulcerative colitis" indicates the serum samples of patients with ulcerative colitis, "Crohn's disease" indicates the serum samples of patients with Crohn's disease, and "Other intestinal diseases" indicates the serum samples of patients with other intestinal diseases. A cut-off value indicated by a broken line is a sum of the average relative absorbance of the serum samples of patients with other intestinal diseases and a value that is 3 times the standard deviation (SD).

Concerning the anti-human integrin αVβ3 antibody, 60 samples out of the 66 serum samples of patients with ulcerative colitis were positive therefor (sensitivity: 91%). In contrast, 38 samples out of the 47 serum samples of patients with Crohn's disease were negative therefor, and 9 samples out of the 9 serum samples of patients with other intestinal diseases were negative therefor (specificity: 84%). The results demonstrate that the concentration of the autoantibody to human integrin αVβ3 in the blood is specifically high in patients with ulcerative colitis.

### <Experiment 12: Diagnosis of ulcerative colitis based on the anti-human integrin αVβ3 antibody>

This experiment confirmed that the method for diagnosis of ulcerative colitis based on the anti-human integrin αVβ3 antibody concentration in the serum would enable specific diagnosis of ulcerative colitis. Samples used in this experiment included: serum samples of patients with ulcerative colitis (n = 51), serum samples of patients with Crohn's disease (n = 26), serum samples of patients with other intestinal diseases (infectious enteritis, ischemic enteritis, Behcet's disease, eosinophilic gastroenteritis, Cronkhite-Canada syndrome, or IBDU) (n = 24), serum samples of patients with collagen disease (n = 27), and serum samples of healthy subjects (n = 22).

In this experiment, also, patients with ulcerative colitis not complicated with primary sclerosing cholangitis were selected.

The anti-human integrin αVβ3 antibody concentration in the serum samples was assayed via ELISA in the same manner as in Experiment 4.

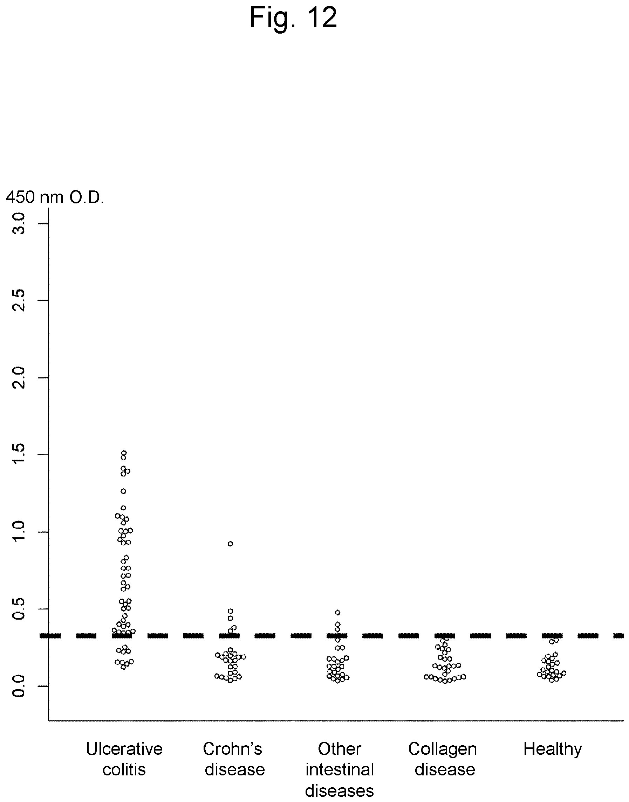

The results are shown in Fig. 12. In Fig. 12, the vertical axis indicates the relative absorbance at 450 nm proportional to the amount of the detection antibody on a solid phase. "UC" indicates the serum samples of patients with ulcerative colitis, "CD" indicates the serum samples of patients with Crohn's disease, "Other intestinal diseases" indicates the serum samples of patients with other intestinal diseases, "Collagen disease" indicates the serum samples of patients with collagen disease, and "Healthy" indicates the serum samples of healthy subjects. A cut-off value indicated by a broken line is a sum of the average relative absorbance of the serum samples of a healthy person and a value that is 3 times the standard deviation (SD).

Concerning the anti-human integrin αVβ3 antibody, 42 samples out of the 51 serum samples of patients with ulcerative colitis were positive therefor (sensitivity: 82%). In contrast, 21 samples out of the 26 serum samples of patients with Crohn's disease were negative therefor, 21 samples out of the 24 serum samples of patients with other intestinal diseases were negative therefor, 27 samples out of the 27 serum samples of patients with collagen disease were negative therefor, and 22 samples out of the 22 serum samples of healthy subjects were negative therefor (specificity: 92%). The results demonstrate that the concentration of the autoantibody to human integrin αVβ3 in the blood is specifically high in patients with ulcerative colitis as in the case of Experiment 11.

### <Experiment 13: IgG subclass of anti-human integrin αVβ3 autoantibody in patient with ulcerative colitis>

This experiment studied the IgG subclasses of the anti-human integrin αVβ3 autoantibody in the serum samples obtained from patients with ulcerative colitis.

Samples used in this experiment included: serum samples of patients with ulcerative colitis (n = 47), serum samples of patients with Crohn's disease (n = 8), and serum samples of patients with other intestinal diseases (infectious enteritis, ischemic enteritis, Behcet's disease, eosinophilic gastroenteritis, Cronkhite-Canada syndrome, or IBDU) (n = 8).

In this experiment, also, patients with ulcerative colitis not complicated with primary sclerosing cholangitis were selected.

In order to detect IgG1, IgG2, IgG3, and IgG4 that specifically bind to human integrin αVβ3 while distinguishing them from one another, the ELISA technique involving the use of human integrin αVβ3 as an antigen described in Experiment 1 was performed in the same manner herein, except that the HRP-conjugated anti-human IgG1 antibody (Anti-IgG1-HRP, AP006, Binding Site), the HRP-conjugated anti-human IgG2 antibody (Anti-IgG2-HRP, AP007, Binding Site), the HRP-conjugated anti-human IgG3 antibody (Anti-IgG3-HRP, AP008, Binding Site), or the HRP-conjugated anti-human IgG4 antibody (Anti-IgG4-HRP, AP009, Binding Site) was used as the detection antibody and CaCl₂ and MgCl₂ were added to the final concentration of 1 mM to the ELISA wash solution, the ELISA blocking buffer, and the conjugate diluent.

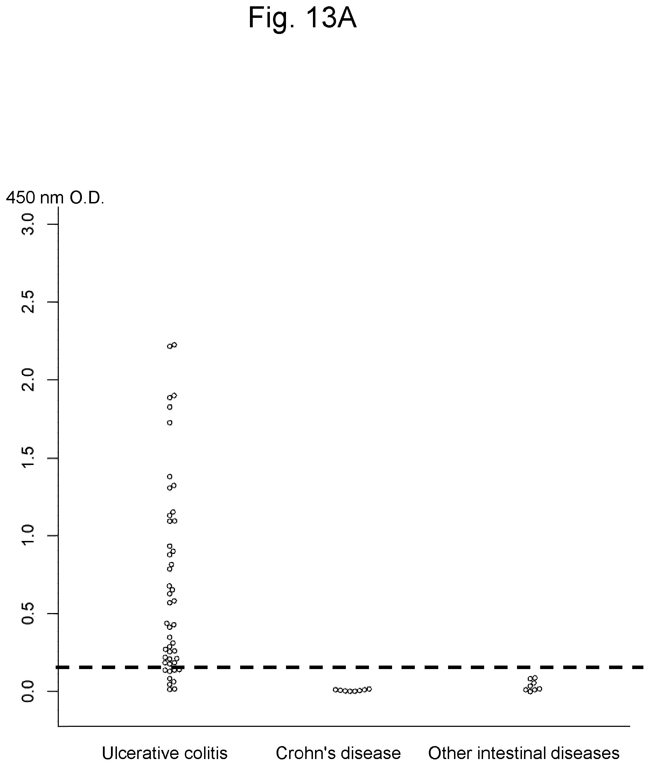

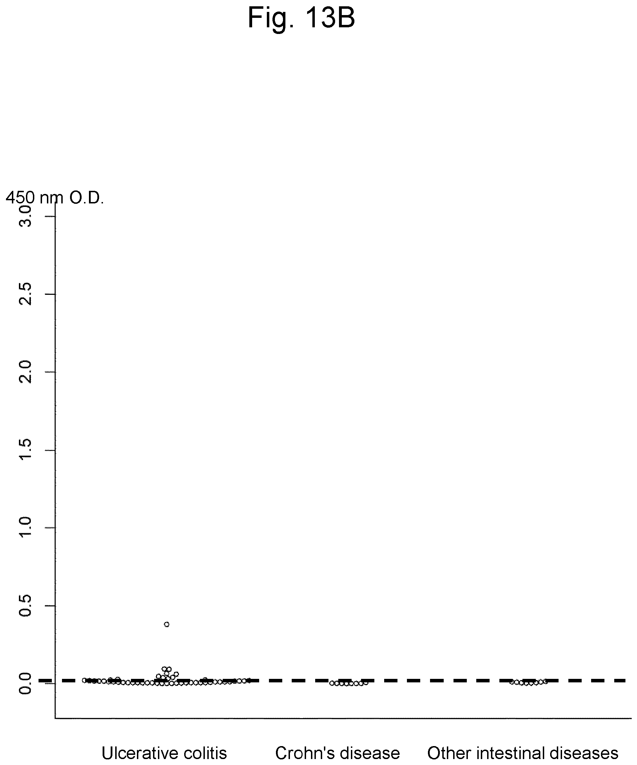

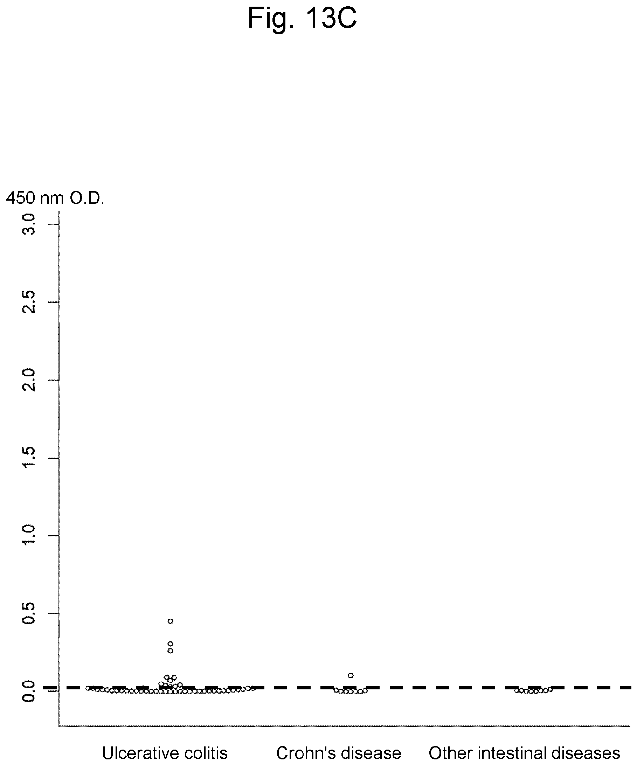

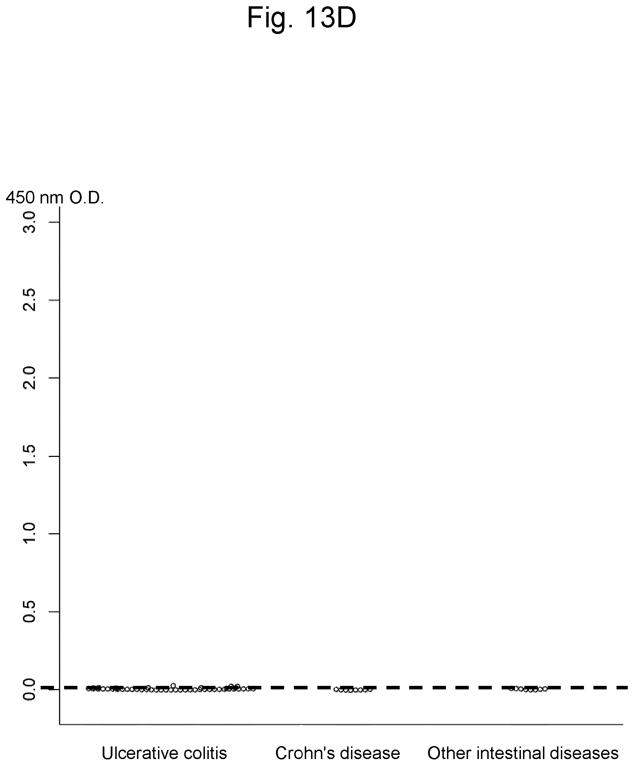

Fig. 13A shows the results of IgG1 detection, Fig. 13B shows the results of IgG2 detection, Fig. 13C shows the results of IgG3 detection, and Fig. 13D shows the results of IgG4 detection. In Fig. 13A to Fig. 13D, the vertical axis indicates the relative absorbance at 450 nm proportional to the amount of the detection antibody on a solid phase. "Ulcerative colitis" indicates the serum samples of patients with ulcerative colitis (n = 47), "Crohn's disease" indicates the serum samples of patients with Crohn's disease (n = 8), and "Other intestinal diseases" indicates the serum samples of patients with other intestinal diseases (n = 8). In Fig. 13A to Fig. 13D, a cut-off value indicated by a broken line is a sum of the average relative absorbance of the serum samples of patients with other intestinal diseases and a value that is 3 times the standard deviation (SD).

Fig. 13A shows the results described below. That is, 39 samples out of the 47 serum samples of patients with ulcerative colitis were positive for IgG1 that binds to human integrin αVβ3 (i.e., sensitivity: 83%), 8 samples out of the 8 serum samples of patients with Crohn's disease were negative therefor, and 8 samples out of the 8 serum samples of patients with other intestinal diseases were negative therefor.

Fig. 13B shows the results described below. That is, 14 samples out of the 47 serum samples of patients with ulcerative colitis were positive for IgG2 that binds to human integrin αVβ3 (i.e., sensitivity: 30%), 8 samples out of the 8 serum samples of patients with Crohn's disease were negative therefor, and 8 samples out of the 8 serum samples of patients with other intestinal diseases were negative therefor.

Fig. 13C shows the results described below. That is, 16 samples out of the 47 serum samples of patients with ulcerative colitis were positive for IgG3 that binds to human integrin αVβ3 (i.e., sensitivity: 34%), 8 samples out of the 8 serum samples of patients with Crohn's disease were negative therefor, and 8 samples out of the 8 serum samples of patients with other intestinal diseases were negative therefor.

Fig. 13D shows the results described below. That is, 3 samples out of the 47 serum samples of patients with ulcerative colitis were positive for IgG4 that binds to human integrin αVβ3 (i.e., sensitivity: 6%), 8 samples out of the 8 serum samples of patients with Crohn's disease were negative therefor, and 8 samples out of the 8 serum samples of patients with other intestinal diseases were negative therefor.

The results of experiment demonstrate that IgG1 is the dominant IgG subclass of the anti-human integrin αVβ3 autoantibody in a patient with ulcerative colitis.

### <Experiment 14: Isotype of the anti-human integrin αVβ3 autoantibody in patient with ulcerative colitis>

In this experiment, isotypes other than IgG; i.e., IgA, IgM, and IgE, were detected as the anti-human integrin αVβ3 autoantibodies in the serum samples of patients with ulcerative colitis.

Samples used in this experiment included: serum samples of patients with ulcerative colitis (n = 47), serum samples of patients with Crohn's disease (n = 8), and serum samples of patients with other intestinal diseases (infectious enteritis, ischemic enteritis, Behcet's disease, eosinophilic gastroenteritis, Cronkhite-Canada syndrome, or IBDU) (n = 8).

In this experiment, also, patients with ulcerative colitis not complicated with primary sclerosing cholangitis were selected.

In order to detect IgA, IgM, and IgE that specifically bind to human integrin αVβ3, the ELISA technique involving the use of human integrin αVβ3 as an antigen described in Experiment 1 was performed in the same manner herein, except that the HRP-conjugated anti-human IgA antibody (Goat anti-Human IgA Antibody HRP Conjugated, A80-102P, BETHYL), the HRP-conjugated anti-human IgM antibody (Goat anti-Human IgM Antibody HRP Conjugated, A80-100P, BETHYL), or the HRP-conjugated anti-human IgE antibody (Goat anti-Human IgE Cross-Adsorbed Secondary Antibody, HRP, A18799, Invitrogen) was used as the detection antibody and CaCl₂ and MgCl₂ were added to the final concentration of 1 mM to the ELISA wash solution, the ELISA blocking buffer, and the conjugate diluent.

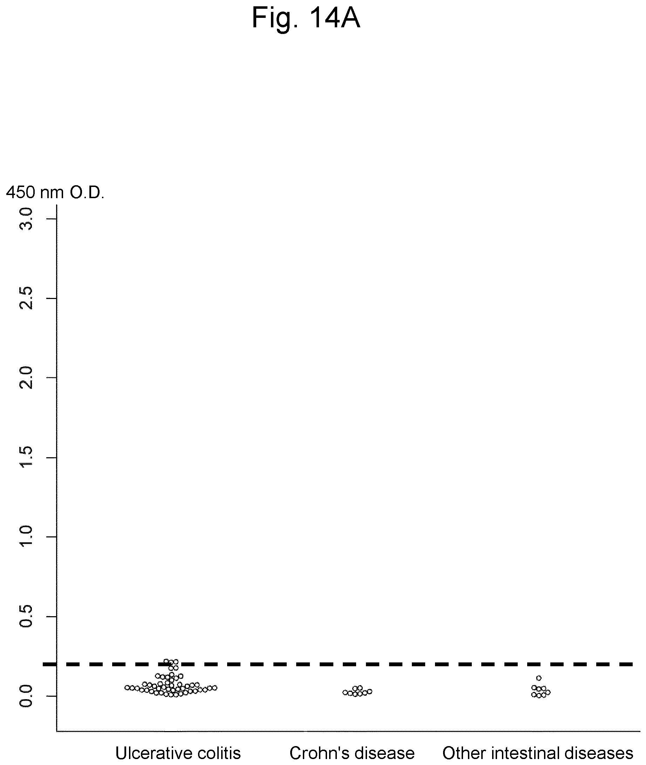

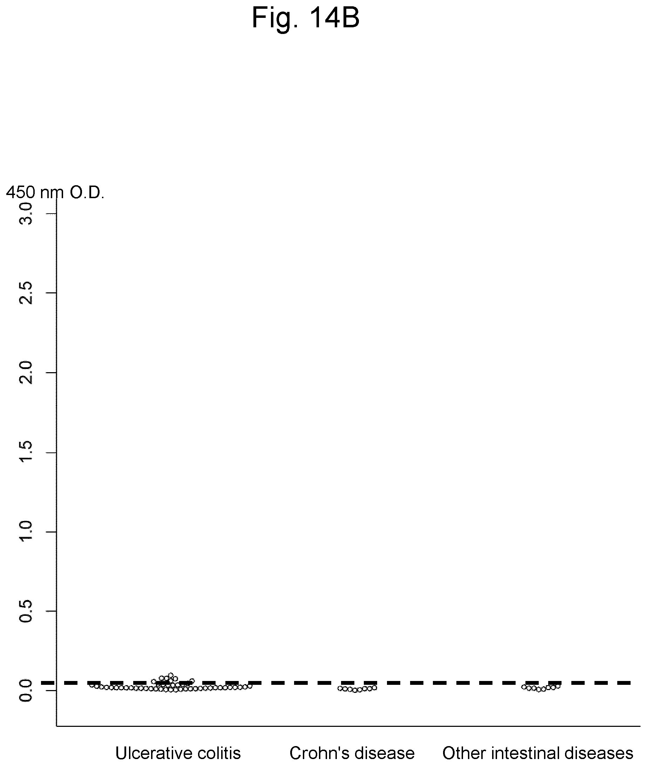

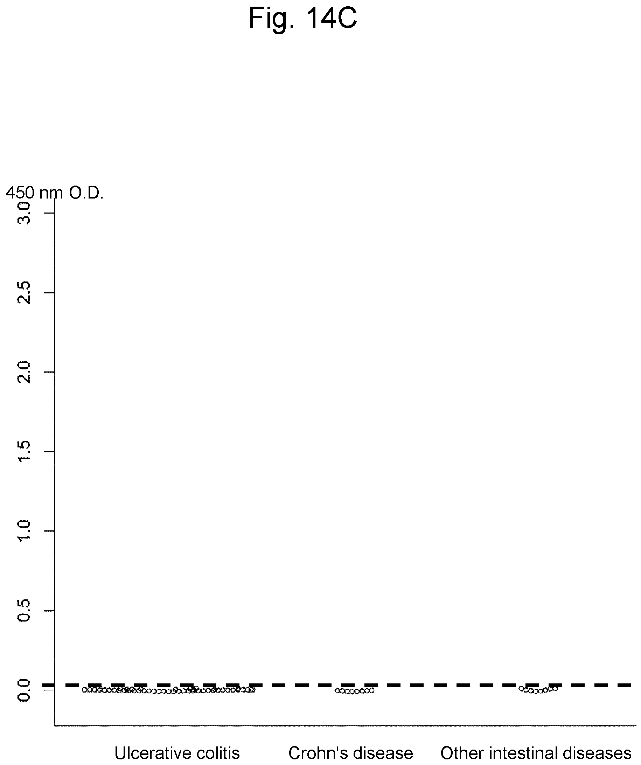

Fig. 14A shows the results of IgA detection, Fig. 14B shows the results of IgM detection, and Fig. 14C shows the results of IgE detection. In Fig. 14A to Fig. 14C, the vertical axis indicates the relative absorbance at 450 nm proportional to the amount of the detection antibody on a solid phase. "Ulcerative colitis" indicates the serum samples of patients with ulcerative colitis (n = 47), "Crohn's disease" indicates the serum samples of patients with Crohn's disease (n = 8), and "Other intestinal diseases" indicates the serum samples of patients with other intestinal diseases (n = 8). In Fig. 14A to Fig. 14C, a cut-off value indicated by a broken line is a sum of the average relative absorbance of the serum samples of patients with other intestinal diseases and a value that is 3 times the standard deviation (SD).

Fig. 14A shows the results described below. That is, 5 samples out of the 47 serum samples of patients with ulcerative colitis were positive for IgA that binds to human integrin αVβ3 (i.e., sensitivity: 11%), 8 samples out of the 8 serum samples of patients with Crohn's disease were negative therefor, and 8 samples out of the 8 serum samples of patients with other intestinal diseases were negative therefor.

Fig. 14B shows the results described below. That is, 10 samples out of the 47 serum samples of patients with ulcerative colitis were positive for IgM that binds to human integrin αVβ3 (i.e., sensitivity: 21%), 8 samples out of the 8 serum samples of patients with Crohn's disease were negative therefor, and 8 samples out of the 8 serum samples of patients with other intestinal diseases were negative therefor.

Fig. 14C shows the results described below. That is, 0 samples out of the 47 serum samples of patients with ulcerative colitis were positive for IgE that binds to human integrin αVβ3 (i.e., sensitivity: 0%), 8 samples out of the 8 serum samples of patients with Crohn's disease were negative therefor, and 8 samples out of the 8 serum samples of patients with other intestinal diseases were negative therefor.

The results of experiment demonstrate that IgA, IgM, and IgE are minor anti-human integrin αVβ3 autoantibody isotypes in patients with ulcerative colitis.

## Claims

1. A method for testing ulcerative colitis comprising:
a step of detection comprising detecting, as an indicator of ulcerative colitis, an antibody immunologically reacting with full-length or a fragment of integrin αVβ6 and/or an antibody immunologically reacting with full-length or a fragment of integrin αVβ3 in a specimen; wherein the specimen is a blood sample, wherein;
a) the fragment of integrin αVβ6 is a dimer composed of an αV chain comprising Phe 31 to Val 992 of the amino acid sequence of the αV chain as shown in SEQ ID NO: 1 and a β6 chain comprising Gly 22 to Asn 707 of the amino acid sequence of the β6 chain as shown in SEQ ID NO: 2, and;
b) the fragment of integrin αVβ3 is a dimer composed of an αV chain comprising Phe 31 to Val 992 of the amino acid sequence of the αV chain as shown in SEQ ID NO: 1 and a β3 chain comprising Gly 27 to Asp 718 of the amino acid sequence of the β3 chain as shown in SEQ ID NO: 3.

2. The method according to Claim 1, wherein the step of detection comprises using full-length or a fragment of integrin αVβ6 as an antigen to detect an antibody immunologically reacting therewith and/or using full-length or a fragment of integrin αVβ3 as an antigen to detect an antibody immunologically reacting therewith.

3. Use of a test kit for testing ulcerative colitis on a blood sample wherein said test kit comprises full-length or a fragment of integrin αVβ6 and/or full-length or a fragment of integrin αVβ3, wherein;
a) the fragment of integrin αVβ6 is a dimer composed of an αV chain comprising Phe 31 to Val 992 of the amino acid sequence of the αV chain as shown in SEQ ID NO: 1 and a β6 chain comprising Gly 22 to Asn 707 of the amino acid sequence of the β6 chain as shown in SEQ ID NO: 2, and;
b) the fragment of integrin αVβ3 is a dimer composed of an αV chain comprising Phe 31 to Val 992 of the amino acid sequence of the αV chain as shown in SEQ ID NO: 1 and a β3 chain comprising Gly 27 to Asp 718 of the amino acid sequence of the β3 chain as shown in SEQ ID NO: 3.

4. Use according to Claim 3, wherein said test kit further comprises a detection antibody.

5. Use according to Claim 3 or 4, wherein said test kit further comprises a positive standard sample solution and/or a negative standard sample solution.

6. Use according to any one of Claims 3 to 5, wherein said test kit comprises the full-length or a fragment of integrin αVβ6 and/or the full-length or a fragment of integrin αVβ3 immobilized on a solid phase.

7. A method for testing primary sclerosing cholangitis comprising:
a step of detection comprising detecting, as an indicator of primary sclerosing cholangitis, an antibody immunologically reacting with full-length or a fragment of integrin αVβ6 and/or an antibody immunologically reacting with full-length or a fragment of integrin αVβ3 in a specimen; wherein the specimen is a blood sample, wherein;
a) the fragment of integrin αVβ6 is a dimer composed of an αV chain comprising Phe 31 to Val 992 of the amino acid sequence of the αV chain as shown in SEQ ID NO: 1 and a β6 chain comprising Gly 22 to Asn 707 of the amino acid sequence of the β6 chain as shown in SEQ ID NO: 2, and;
b) the fragment of integrin αVβ3 is a dimer composed of an αV chain comprising Phe 31 to Val 992 of the amino acid sequence of the αV chain as shown in SEQ ID NO: 1 and a β3 chain comprising Gly 27 to Asp 718 of the amino acid sequence of the β3 chain as shown in SEQ ID NO: 3.

8. The method according to Claim 7, wherein the step of detection comprises using full-length or a fragment of integrin αVβ6 as an antigen to detect an antibody immunologically reacting therewith and/or using full-length or a fragment of integrin αVβ3 as an antigen to detect an antibody immunologically reacting therewith.

9. Use of a test kit for testing primary sclerosing cholangitis on a blood sample wherein said test kit comprises full-length or a fragment of integrin αVβ6 and/or full-length or a fragment of integrin αVβ3, wherein;
a) the fragment of integrin αVβ6 is a dimer composed of an αV chain comprising Phe 31 to Val 992 of the amino acid sequence of the αV chain as shown in SEQ ID NO: 1 and a β6 chain comprising Gly 22 to Asn 707 of the amino acid sequence of the β6 chain as shown in SEQ ID NO: 2, and;
b) the fragment of integrin αVβ3 is a dimer composed of an αV chain comprising Phe 31 to Val 992 of the amino acid sequence of the αV chain as shown in SEQ ID NO: 1 and a β3 chain comprising Gly 27 to Asp 718 of the amino acid sequence of the β3 chain as shown in SEQ ID NO: 3.

10. Use according to Claim 9, wherein said test kit further comprises a detection antibody.

11. Use according to Claim 9 or 10, wherein said test kit further comprises a positive standard sample solution and/or a negative standard sample solution.

12. Use according to any one of Claims 9 to 11, wherein said test kit comprises the full-length or a fragment of integrin αVβ6 and/or the full-length or a fragment of integrin αVβ3 immobilized on a solid phase.

## Patentansprüche

1. Verfahren zum Testen auf Colitis ulcerosa, umfassend:
einen Nachweisschritt, umfassend das Nachweisen, als Indikator für Colitis ulcerosa, eines Antikörpers, der immunologisch mit Volllängenform oder einem Fragment von Integrin αVβ6 reagiert, und/oder eines Antikörpers, der immunologisch mit Volllängenform oder einem Fragment von Integrin αVβ3 reagiert, in einer Testprobe; wobei es sich bei der Testprobe um eine Blutprobe handelt, wobei
a) es sich bei dem Fragment von Integrin αVβ6 um ein Dimer handelt, das aus einer αV-Kette, die Phe 31 bis Val 992 der Aminosäuresequenz der αV-Kette gemäß SEQ ID NO: 1 umfasst, und einer β6-Kette, die Gly 22 bis Asn 707 der Aminosäuresequenz der β6-Kette gemäß SEQ ID NO: 2 umfasst, zusammengesetzt ist, und
b) es sich bei dem Fragment von Integrin αVβ3 um ein Dimer handelt, das aus einer αV-Kette, die Phe 31 bis Val 992 der Aminosäuresequenz der αV-Kette gemäß SEQ ID NO: 1 umfasst, und einer β3-Kette, die Gly 27 bis Asp 718 der Aminosäuresequenz der β3-Kette gemäß SEQ ID NO: 3 umfasst, zusammengesetzt ist.

2. Verfahren nach Anspruch 1, wobei der Nachweisschritt das Verwenden von Volllängenform oder einem Fragment von Integrin αVβ6 als Antigen zum Nachweisen eines Antikörpers, der immunologisch damit reagiert, und/oder das Verwenden von Volllängenform oder einem Fragment von Integrin αVβ3 als Antigen zum Nachweisen eines Antikörpers, der immunologisch damit reagiert, umfasst.

3. Verwendung eines Testkits zum Testen auf Colitis ulcerosa anhand einer Blutprobe, wobei das Testkit Volllängenform oder ein Fragment von Integrin αVβ6 und/oder Volllängenform oder ein Fragment von Integrin αVβ3 umfasst, wobei
a) es sich bei dem Fragment von Integrin αVβ6 um ein Dimer handelt, das aus einer αV-Kette, die Phe 31 bis Val 992 der Aminosäuresequenz der αV-Kette gemäß SEQ ID NO: 1 umfasst, und einer β6-Kette, die Gly 22 bis Asn 707 der Aminosäuresequenz der β6-Kette gemäß SEQ ID NO: 2 umfasst, zusammengesetzt ist, und
b) es sich bei dem Fragment von Integrin αVβ3 um ein Dimer handelt, das aus einer αV-Kette, die Phe 31 bis Val 992 der Aminosäuresequenz der αV-Kette gemäß SEQ ID NO: 1 umfasst, und einer β3-Kette, die Gly 27 bis Asp 718 der Aminosäuresequenz der β3-Kette gemäß SEQ ID NO: 3 umfasst, zusammengesetzt ist.

4. Verwendung nach Anspruch 3, wobei das Testkit ferner einen Nachweisantikörper umfasst.

5. Verwendung nach Anspruch 3 oder 4, wobei das Testkit ferner eine Positivstandard-Probenlösung und/oder eine Negativstandard-Probenlösung umfasst.

6. Verwendung nach einem der Ansprüche 3 bis 5, wobei das Testkit die Volllängenform oder ein Fragment von Integrin αVβ6 und/oder die Volllängenform oder ein Fragment von Integrin αVβ3, immobilisiert auf einer festen Phase, umfasst.

7. Verfahren zum Testen auf primär sklerosierende Cholangitis, umfassend:
einen Nachweisschritt, umfassend das Nachweisen, als Indikator für primär sklerosierende Cholangitis, eines Antikörpers, der immunologisch mit Volllängenform oder einem Fragment von Integrin αVβ6 reagiert, und/oder eines Antikörpers, der immunologisch mit Volllängenform oder einem Fragment von Integrin αVβ3 reagiert, in einer Testprobe; wobei es sich bei der Testprobe um eine Blutprobe handelt, wobei
a) es sich bei dem Fragment von Integrin αVβ6 um ein Dimer handelt, das aus einer αV-Kette, die Phe 31 bis Val 992 der Aminosäuresequenz der αV-Kette gemäß SEQ ID NO: 1 umfasst, und einer β6-Kette, die Gly 22 bis Asn 707 der Aminosäuresequenz der β6-Kette gemäß SEQ ID NO: 2 umfasst, zusammengesetzt ist, und
b) es sich bei dem Fragment von Integrin αVβ3 um ein Dimer handelt, das aus einer αV-Kette, die Phe 31 bis Val 992 der Aminosäuresequenz der αV-Kette gemäß SEQ ID NO: 1 umfasst, und einer β3-Kette, die Gly 27 bis Asp 718 der Aminosäuresequenz der β3-Kette gemäß SEQ ID NO: 3 umfasst, zusammengesetzt ist.

8. Verfahren nach Anspruch 7, wobei der Nachweisschritt das Verwenden von Volllängenform oder einem Fragment von Integrin αVβ6 als Antigen zum Nachweisen eines Antikörpers, der immunologisch damit reagiert, und/oder das Verwenden von Volllängenform oder einem Fragment von Integrin αVβ3 als Antigen zum Nachweisen eines Antikörpers, der immunologisch damit reagiert, umfasst.

9. Verwendung eines Testkits zum Testen auf primär sklerosierende Cholangitis anhand einer Blutprobe, wobei das Testkit Volllängenform oder ein Fragment von Integrin αVβ6 und/oder Volllängenform oder ein Fragment von Integrin αVβ3 umfasst, wobei
a) es sich bei dem Fragment von Integrin αVβ6 um ein Dimer handelt, das aus einer αV-Kette, die Phe 31 bis Val 992 der Aminosäuresequenz der αV-Kette gemäß SEQ ID NO: 1 umfasst, und einer β6-Kette, die Gly 22 bis Asn 707 der Aminosäuresequenz der β6-Kette gemäß SEQ ID NO: 2 umfasst, zusammengesetzt ist, und
b) es sich bei dem Fragment von Integrin αVβ3 um ein Dimer handelt, das aus einer αV-Kette, die Phe 31 bis Val 992 der Aminosäuresequenz der αV-Kette gemäß SEQ ID NO: 1 umfasst, und einer β3-Kette, die Gly 27 bis Asp 718 der Aminosäuresequenz der β3-Kette gemäß SEQ ID NO: 3 umfasst, zusammengesetzt ist.

10. Verwendung nach Anspruch 9, wobei das Testkit ferner einen Nachweisantikörper umfasst.

11. Verwendung nach Anspruch 9 oder 10, wobei das Testkit ferner eine Positivstandard-Probenlösung und/oder eine Negativstandard-Probenlösung umfasst.

12. Verwendung nach einem der Ansprüche 9 bis 11, wobei das Testkit die Volllängenform oder ein Fragment von Integrin αVβ6 und/oder die Volllängenform oder ein Fragment von Integrin αVβ3, immobilisiert auf einer festen Phase, umfasst.

## Revendications

1. Méthode de dépistage de la rectocolite hémorragique comprenant :
une étape de détection comprenant la détection, en tant qu'indicateur de la rectocolite hémorragique, d'un anticorps réagissant immunologiquement avec l'intégrine αVβ6 pleine longueur ou un fragment de celle-ci et/ou d'un anticorps réagissant immunologiquement avec l'intégrine αVβ3 pleine longueur ou un fragment de celle-ci dans un échantillon ; l'échantillon étant un échantillon de sang, dans laquelle ;
a) le fragment d'intégrine αVβ6 est un dimère composé d'une chaîne αV comprenant Phe 31 à Val 992 de la séquence d'acides aminés de la chaîne αV comme montré dans SEQ ID NO: 1 et une chaîne β6 comprenant Gly 22 à Asn 707 de la séquence d'acides aminés de la chaîne β6 comme montré dans SEQ ID NO: 2, et ;
b) le fragment d'intégrine αVβ3 est un dimère composé d'une chaîne αV comprenant Phe 31 à Val 992 de la séquence d'acides aminés de la chaîne αV comme montré dans SEQ ID NO: 1 et une chaîne β3 comprenant Gly 27 à Asp 718 de la séquence d'acides aminés de la chaîne β3 comme montré dans SEQ ID NO: 3.

2. Méthode selon la revendication 1, dans laquelle l'étape de détection comprend l'utilisation de l'intégrine αVβ6 pleine longueur ou un fragment de celle-ci comme antigène pour détecter un anticorps réagissant immunologiquement avec celle-ci et/ou l'utilisation de l'intégrine αVβ3 pleine longueur ou un fragment de celle-ci comme antigène pour détecter un anticorps réagissant immunologiquement avec celle-ci.

3. Utilisation d'un kit de dépistage pour détecter la rectocolite hémorragique sur un échantillon de sang, ledit kit de dépistage comprenant l'intégrine αVβ6 pleine longueur ou un fragment de celle-ci et/ou l'intégrine αVβ3 pleine longueur ou un fragment de celle-ci, dans laquelle ;
a) le fragment d'intégrine αVβ6 est un dimère composé d'une chaîne αV comprenant Phe 31 à Val 992 de la séquence d'acides aminés de la chaîne αV comme montré dans SEQ ID NO: 1 et une chaîne β6 comprenant Gly 22 à Asn 707 de la séquence d'acides aminés de la chaîne β6 comme montré dans SEQ ID NO: 2, et ;
b) le fragment d'intégrine αVβ3 est un dimère composé d'une chaîne αV comprenant Phe 31 à Val 992 de la séquence d'acides aminés de la chaîne αV comme montré dans SEQ ID NO: 1 et une chaîne β3 comprenant Gly 27 à Asp 718 de la séquence d'acides aminés de la chaîne β3 comme montré dans SEQ ID NO: 3.

4. Utilisation selon la revendication 3, ledit kit de dépistage comprenant en outre un anticorps de détection.

5. Utilisation selon la revendication 3 ou 4, ledit kit de dépistage comprenant en outre une solution d'échantillon témoin positif et/ou une solution d'échantillon témoin négatif.

6. Utilisation selon l'une quelconque des revendications 3 à 5, ledit kit de dépistage comprenant l'intégrine αVβ6 pleine longueur ou un fragment de celle-ci et/ou l'intégrine αVβ3 pleine longueur ou un fragment de celle-ci immobilisés sur une phase solide.

7. Méthode de dépistage de la cholangite sclérosante primitive comprenant :
une étape de détection comprenant la détection, en tant qu'indicateur de la cholangite sclérosante primitive, d'un anticorps réagissant immunologiquement avec l'intégrine αVβ6 pleine longueur ou un fragment de celle-ci et/ou d'un anticorps réagissant immunologiquement avec l'intégrine αVβ3 pleine longueur ou un fragment de celle-ci dans un échantillon ; l'échantillon étant un échantillon de sang, dans laquelle ;
a) le fragment d'intégrine αVβ6 est un dimère composé d'une chaîne αV comprenant Phe 31 à Val 992 de la séquence d'acides aminés de la chaîne αV comme montré dans SEQ ID NO: 1 et une chaîne β6 comprenant Gly 22 à Asn 707 de la séquence d'acides aminés de la chaîne β6 comme montré dans SEQ ID NO: 2, et ;
b) le fragment d'intégrine αVβ3 est un dimère composé d'une chaîne αV comprenant Phe 31 à Val 992 de la séquence d'acides aminés de la chaîne αV comme montré dans SEQ ID NO: 1 et une chaîne β3 comprenant Gly 27 à Asp 718 de la séquence d'acides aminés de la chaîne β3 comme montré dans SEQ ID NO: 3.

8. Méthode selon la revendication 7, dans laquelle l'étape de détection comprend l'utilisation de l'intégrine αVβ6 pleine longueur ou un fragment de celle-ci comme antigène pour détecter un anticorps réagissant immunologiquement avec celle-ci et/ou l'utilisation de l'intégrine αVβ3 pleine longueur ou un fragment de celle-ci comme antigène pour détecter un anticorps réagissant immunologiquement avec celle-ci.

9. Utilisation d'un kit de dépistage pour détecter la cholangite sclérosante primitive sur un échantillon de sang, ledit kit de dépistage comprenant l'intégrine αVβ6 pleine longueur ou un fragment de celle-ci et/ou l'intégrine αVβ3 pleine longueur ou un fragment de celle-ci, dans laquelle ;
a) le fragment d'intégrine αVβ6 est un dimère composé d'une chaîne αV comprenant Phe 31 à Val 992 de la séquence d'acides aminés de la chaîne αV comme montré dans SEQ ID NO: 1 et une chaîne β6 comprenant Gly 22 à Asn 707 de la séquence d'acides aminés de la chaîne β6 comme montré dans SEQ ID NO: 2, et ;
b) le fragment d'intégrine αVβ3 est un dimère composé d'une chaîne αV comprenant Phe 31 à Val 992 de la séquence d'acides aminés de la chaîne αV comme montré dans SEQ ID NO: 1 et une chaîne β3 comprenant Gly 27 à Asp 718 de la séquence d'acides aminés de la chaîne β3 comme montré dans SEQ ID NO: 3.

10. Utilisation selon la revendication 9, ledit kit de dépistage comprenant en outre un anticorps de détection.

11. Utilisation selon la revendication 9 ou 10, ledit kit de dépistage comprenant en outre une solution d'échantillon témoin positif et/ou une solution d'échantillon témoin négatif.

12. Utilisation selon l'une quelconque des revendications 9 à 11, ledit kit de dépistage comprenant l'intégrine αVβ6 pleine longueur ou un fragment de celle-ci et/ou l'intégrine αVβ3 pleine longueur ou un fragment de celle-ci immobilisés sur une phase solide.
